# EUROPEAN PATENT APPLICATION

(11) **EP 4 599 885 A2**
(43) Date of publication of application: **13.08.2025**
(21) Application number: 25168445.2
(22) Date of filing: 02.06.2022
(51) Int. Cl.: A61N 5/10

(54) **COMPOUNDS FOR THE TREATMENT OF GLIOBLASTOMA**

(30) Priority: 04.06.2021 US 202163197163 P; 04.10.2021 US 202163251945 P; 12.11.2021 US 202163278789 P
(62) Divisional of application: 22732481.1
(71) Applicant: Merck Patent GmbH, 64293 Darmstadt (DE)
(72) Inventor: GANN, Claudia-Nanette, 64293 Darmstadt (DE); CELIK, Ilhan, 64293 Darmstadt (DE); MAJD, Nazanin, HOUSTON, TX 77030 (US); DE GROOT, John Frederick, SAN FRANCISCO, CA 94143-0350 (US); WELLER, Michael, 8091 ZUERICH (CH)
(74) Representative: Merck Patent Association

(57) **Abstract**

The present invention encompasses cMET inhibitors for use in the treatment of glioblastoma, including disseminated glioblastoma harboring MET amplification.

## Description

### TECHNICAL FIELD OF THE INVENTION

The present invention provides for the use of inhibitors of the receptor tyrosine kinase (TKIs) mesenchymal-epithelial transition factor (cMET) in the treatment of glioblastoma, including disseminated glioblastoma harboring MET amplification.

### BACKGROUND OF THE INVENTION

### cMET inhibitors

The mesenchymal-epithelial transition factor (cMet) has emerged as a promising target in the development of anti-cancer therapeutics because of its low level of expression in normal tissues and its aberrant activation in many human cancers. The abnormal stimulation of the multiple signal transduction pathways downstream of the receptor tyrosine kinase cMET promotes cellular transformation, tumor motility, and invasion. Therefore, cMET has been the focus of prognostic and therapeutic studies in different tumor types, including non-small cell lung cancer. In particular, several cMET inhibitors have been developed as innovative therapeutic candidates and are currently under investigation in clinical trials (see e.g., van der Stee et al., 2016, Thoracic One 11(9): 1423). As for selectivity, there is a distinction between type I and II class c-Met inhibitors depending on the binding site on the kinase. Type I inhibitors can be further divided into type Ia and type Ib, with type Ib inhibitors being more highly specific for MET and having fewer off-target effects when compared with type Ia inhibitors. Type II inhibitors inhibit non-activated MET and generally exhibit more off-target effects of other protein kinases, which can cause serious toxic effects. Tepotinib is a highly selective blood-brain-barrier penetrant oral c-MET inhibitor whose hydrochloride hydrate was recently FDA approved for MET-exon-14 altered non-small cell lung cancer (NSCLC). 3-(1-{3-[5-(1-Methyl-piperidin-4-ylmethoxy)-pyrimidin-2-yl]-benzyl}-6-oxo-1,6-dihydro-pyridazin-3-yl)-benzonitrile (tepotinib) was described in WO 2009/006959.

### Glioblastoma

A glioma is a type of tumor that starts in the glial cells of the brain or the spin. Gliomas have been classified by cell type, grade, and location. High-grade gliomas (i.e., WHO grades III-IV) are undifferentiated or anaplastic, malignant and carry a worse prognosis. This group comprises anaplastic astrocytomas and glioblastomas. Glioblastoma is also known as glioblastoma multiforme (GBM). The grading of gliomas changed recently, and glioblastoma is now mainly classified according to the status of isocitrate dehydrogenase (IDH) mutation: IDH-wildtype or IDH-mutant. Glioblastoma is an intrinsic malignant brain tumor probably derived from neuroglial progenitor cells characterized morphologically by invasive growth, focal necrosis, and angiogenesis and on the molecular level by frequent genetic alterations involving the receptor tyrosine kinase (RTK)/RAS/phosphatidylinositol 3-kinase (PI3K), p53 and retinoblastoma (RB) pathways. MET oncogene encodes a receptor-tyrosine-kinase which drives cell proliferation, invasion, and angiogenesis in several solid cancers, including glioblastoma. Pathologic copy-number amplification and point mutation of MET oncologic are drivers in glioblastoma, in which the incidence of MET amplification is 2-5%. MET activation promotes tumor growth, angiogenesis, and invasion, and it is associated with poorer prognosis in gliomas due to poor response to treatment, shorter progression free survival, and overall survival. Tumor growth inhibition and regression of tumors by tepotinib was temporarily observed in mice bearing ligand-independent Hs746T and HGF-dependent U87MG tumors before the tumor became resistant and progressed (Bladt et al., 2013, Clin Cancer Res 19(11): 2941). A combination of tepotinib and 4-[(S)-2-azetidin-1-yl-1-(4-chloro-3-trifluoromethyl-phenyl)-ethylamino]-quinazoline-8-carboxylic acid amide for treating cancer, amongst others glioblastoma in a mouse model, was described in WO 2016/091347.

While there are therapies being considered for use in treatment of glioblastoma, the clinical response is not satisfying. Glioblastoma remains to be one of the most lethal solid cancers. Since the introduction of temozolomide, no other pharmacological treatment has been shown to prolong survival when added to the standard of care of surgery followed by radiotherapy. Testing efficacy of the HGF antibody AMG-102 (Wen et al., 2011, Neuro-oncology 13(4): 437) or the MET antibody onartuzumab (Cloughesy et al., 2017, Journal of Clinical Oncology: Official Journal of the American Society of Clinical Oncology 35(3): 343) had negative outcomes in glioblastoma. Thus, there remains a need to develop novel therapeutic options for the treatment of glioblastoma. Furthermore, there is a need for therapies having greater efficacy than existing therapies.

### BRIEF DESCRIPTION OF THE DRAWINGS

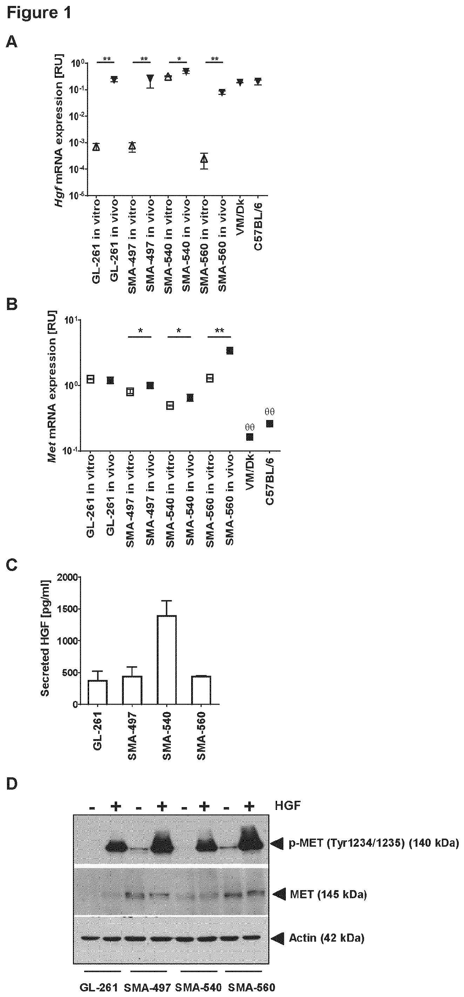

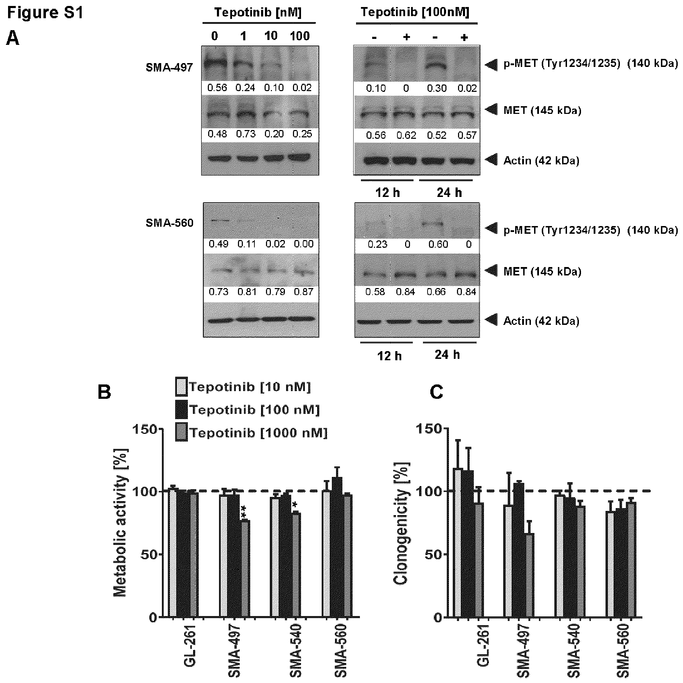

Figure 1 shows HGF/MET pathway activity in murine glioma cells. A, B. *Hgf* and *Met* mRNA expression were assessed by RT-PCR *in vitro* and *ex vivo.* C, D. HGF protein release and constitutive or HGF-stimulated p-MET levels were assessed by ELISA (HGF) (C) or immunoblot (p-MET) *in vitro* (D).

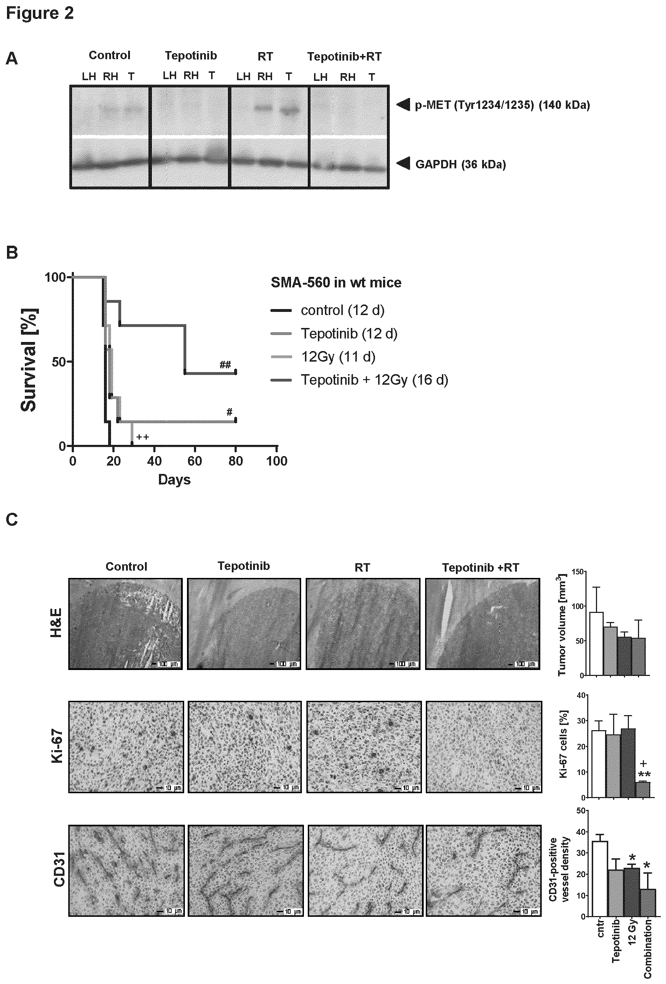

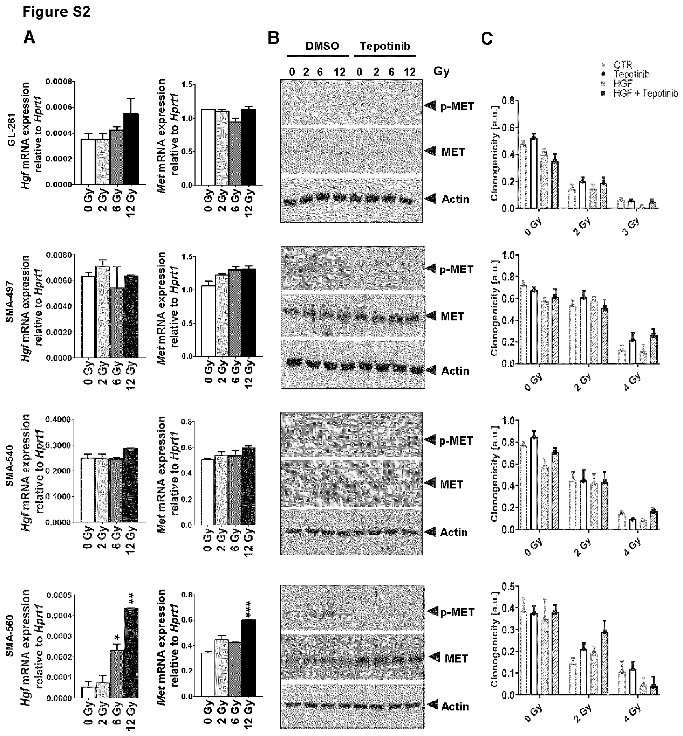

Figure 2 shows synergistic prolongation of survival by irradiation and tepotinib-mediated MET inhibition in the SMA-560 glioma model *in vivo.* A-D. Syngeneic mice were intracranially implanted with SMA-560 glioma cells and treated daily with 100 mg/kg tepotinib from day 6 on or solvent, or with a single dose of 12 Gy on day 7, or in combination. Brains from animals euthanized when the first mice became symptomatic were studied in A and C. A. Tumor lysates pooled from 2 pre-randomized animals per group were analysed by immunoblot for target inhibition (p-MET). B. Kaplan-Meier survival curve. C. Tumor sections from three pre-randomized animals per group were stained for H&E (upper row), Ki-67 (middle row) or CD31 (lower row). Sections were counterstained with hematoxylin (blue). Quantification of immunoreactivity (right panels) (n=3, **p*<0.05, t-test).

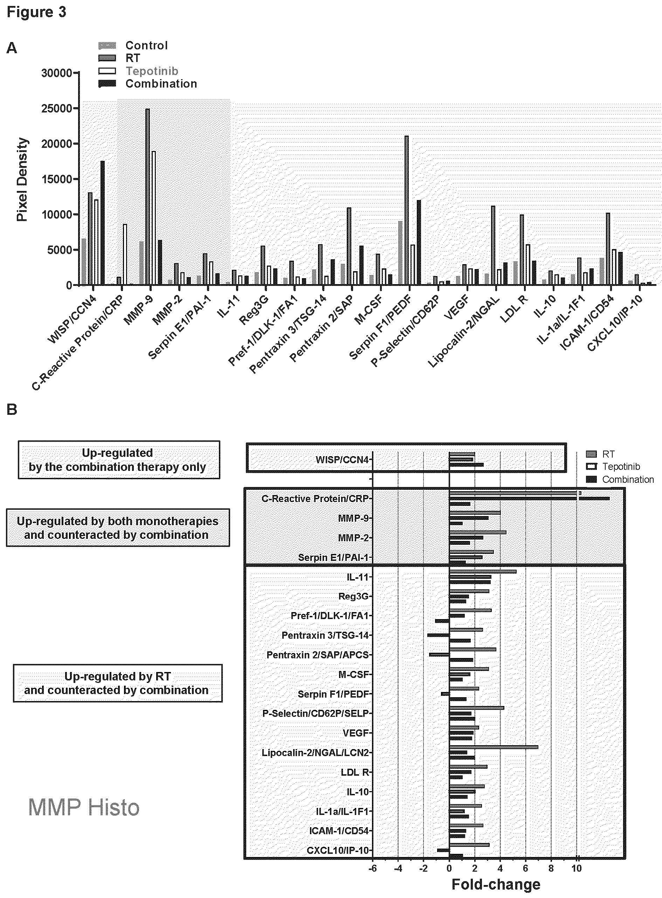

Figure 3 shows modulation of cytokine levels in response to tepotinib, irradiation or combination therapy. Tumor lysates pooled from 2 pre-randomized animals per group were analysed by proteome profiler array. In A, raw data are expressed as pixel density measured by Image J. In B, fold-changes indicate down-regulation (left) or up-regulation (right) versus control tumors. A difference down or up of 2-fold *versus* control was used as cut-off to assign a target to any group.

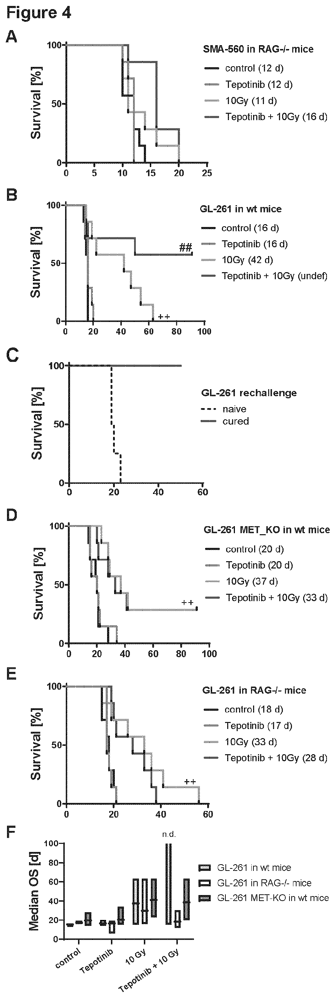

Figure 4 shows modulation of response to tepotinib and irradiation alone or in combination by loss of adaptive immunity. A. *Rag1-*/*-* mice were intracranially implanted with SMA-560 glioma cells, treated daily with 100 mg/kg tepotinib from day 6 on or solvent, or with a single dose of 10 Gy on day 7, or in combination. Kaplan-Meier survival curves are shown. B, C. Syngeneic mice were intracranially implanted with GL-261 glioma cells and treated daily with 100 mg/kg tepotinib from day 6 on or solvent, or with a single dose of 10 Gy on day 7, or in combination. B. Kaplan-Meier survival curves were analyzed via log-rank test. C. Mice surviving in (B) were re-challenged after 13 weeks after initial tumor cell injection with GL-261 cell implantation into the contralateral hemisphere. Naive mice implanted with GL-261 cells were used as controls. Kaplan-Meier survival curves are shown. D, E. GL-261 *met* knock-out cells were intracranially implanted into wildtype C57/Bl6 mice (D) or GL-261 wildtype cells were intracranially implanted into C57/Bl6 *Rag1-*/*-* mice (E). Mice were treated daily with 100 mg/kg tepotinib or solvent from day 6 on, or with a single dose of 10 Gy on day 7, or a combination thereof, and Kaplan-Meier survival curves are shown. F. Analysis of median survival between treatment groups.
Figure S1 shows suppression of MET phosphorylation, but no cytotoxic effects of tepotinib in mouse glioma cell lines. A. SMA-497 or SMA-560 cells were exposed to tepotinib to determine time- and concentration-dependent inhibition of MET phosphorylation. B, C. The cells were exposed to tepotinib in acute growth inhibition (B) or clonogenic survival (C) assays. Viability was assessed by MTT assay (n=3, mean and SEM).
Figure S2 shows modulation of the HGF/MET pathway by irradiation in vitro. A, B. The cells were untreated or exposed to irradiation, and then assessed for expression of *Hgf* and *Met* mRNA (A) or for MET and p-MET levels by immunoblot (B). C. Cells were exposed to irradiation in the absence or presence of tepotinib at 100 nM and/or HGF at 50 ng/ml and were monitored for clonogenic survival (n=6, mean and SEM).

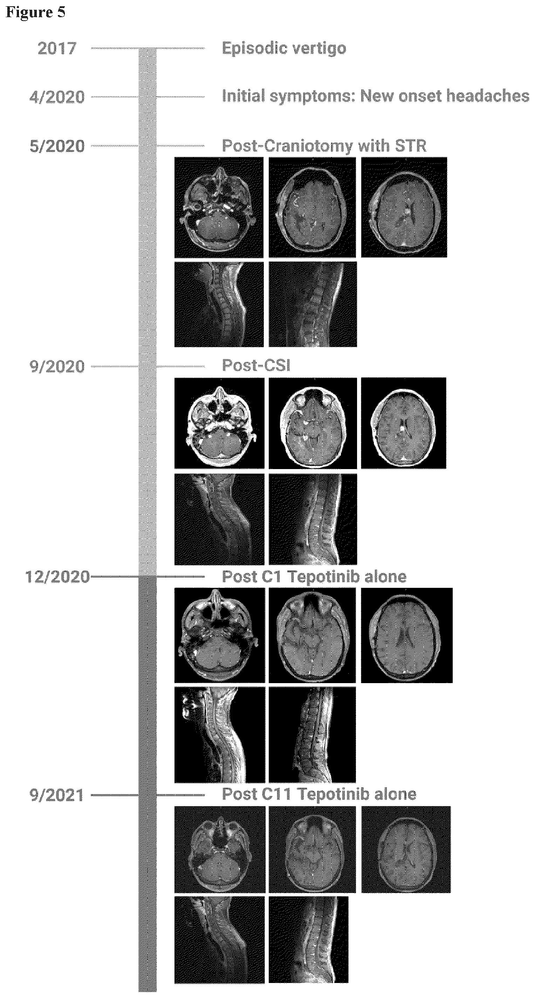

Figure 5 shows the treatment course of a patient and includes MRI screenshots of various intraparenchymal, intraventricular, and spinal cord lesion that were present prior to treatment with tepotinib.

### SUMMARY OF THE INVENTION

Each of the embodiments described below can be combined with any other embodiment described herein not inconsistent with the embodiment with which it is combined. Furthermore, each of the embodiments described herein envisions within its scope pharmaceutically acceptable salts, solvates and/or hydrates of the compounds described herein. Accordingly, the phrase "or a pharmaceutically acceptable salt, solvate and/or hydrate thereof" is implicit in the description of all compounds described herein. Embodiments within an aspect as described below can be combined with any other embodiments not inconsistent within the same aspect or a different aspect.

In a first aspect, the invention provides cMET inhibitors for use in the treatment of glioblastoma in a subject in need thereof. The cMET inhibitor is administered in an amount that is effective in treating a glioblastoma. In one aspect of this embodiment, the cMET inhibitors is used in the treatment of a human subject. In one aspect of this embodiment, the cMET inhibitor is used for long-term treatment and administered for at least 10 weeks. In one aspect of this embodiment, the cMET inhibitor is used for long-term treatment and administered for at least 10 weeks in a line of treatment. In one aspect of this embodiment, the cMET inhibitor for use in the treatment of glioblastoma is not administered in combination with chemotherapy in the same line of treatment. In one aspect of this embodiment, the cMET inhibitor for use in the treatment of glioblastoma is administered in combination with radiotherapy in the same line of treatment. In one embodiment, the radiotherapy is administered as single irradiation dose or after first administration of the cMET inhibitor, or a combination thereof. In one aspect of this embodiment, the glioblastoma is disseminated glioblastoma. In another aspect of this embodiment, the glioblastoma harbors MET amplification. In a further aspect of this embodiment, the glioblastoma harbors IDH wild-type, MGMT-unmethylated and/or MET amplification at 7q31.2. In a final aspect of this embodiment, the cMET inhibitor is tepotinib or a pharmaceutically acceptable salt, solvate and/or hydrate thereof.

A second aspect is a method of treating a glioblastoma in a subject in need thereof, comprising administering an effective amount of a cMET inhibitor, or a pharmaceutically acceptable salt, solvate and/or hydrate thereof, to the subject. In one aspect of this embodiment, the subject to be treated is human. In one aspect of this embodiment, the cMET inhibitor is suitable for long-term treatment and administered for at least 10 weeks. In one aspect of this embodiment, the cMET inhibitor is suitable for long-term treatment and administered for at least 10 weeks in a line of treatment. In one aspect of this embodiment, the cMET inhibitor is not administered in combination with chemotherapy in the same line of treatment. In one aspect of this embodiment, the administration of the cMET inhibitor results in a complete response, increases the likelihood of complete response, reduces the likelihood of development of drug resistance, extends the time to tumor progression and/or extends the time to tumor recurrence in the subject. Also provided are methods of inhibiting tumor growth or progression in a subject who has malignant cells. Also provided are methods of inhibiting metastasis of malignant cells in a subject. Also provided are methods of inducing tumor regression in a subject who has malignant cells. The treatment results in an objective response, preferably a complete response or partial response in the subject. In one aspect of this embodiment, the method further comprises administering radiotherapy in the same line of treatment, optionally as single irradiation dose or after first administration of the cMET inhibitor, or a combination thereof. In one aspect of this embodiment, the cMET inhibitor is administered in a first-line treatment of the glioblastoma. In another aspect of this embodiment, the cMET inhibitor is administered in a second-line treatment or higher line of treatment of the glioblastoma. The therapy of the invention can be used in the glioblastoma treatment of a subject which has been previously treated with one or more chemo- or immunotherapies, or underwent radiotherapy, but failed with any such previous treatment. In a further aspect of this embodiment, the subject is administered a treatment selected from the group of surgery, radiotherapy, chemotherapy, and a combination thereof, prior to first administration of the cMET inhibitor. In a further aspect of this embodiment, the subject is administered a treatment selected from the group of surgery, radiotherapy, or a combination thereof, optionally followed by chemotherapy, prior to first administration of the cMET inhibitor. The radiotherapy can be a treatment given with electrons, photons, protons, alfa-emitters, other ions, radio-nucleotides, boron capture neutrons and combinations thereof. In an additional aspect of this embodiment, the subject is administered tepotinib or a pharmaceutically acceptable salt, solvate and/or hydrate thereof. In a final aspect of this embodiment, the cMET inhibitor is administered as monotherapy.

A third aspect relates to the use of a cMET inhibitor for the manufacture of a medicament for the treatment of glioblastoma. In one aspect of this embodiment, the cMET inhibitor is used for the manufacture of a medicament for the long-term treatment and administered for at least 10 weeks. In one aspect of this embodiment, the cMET inhibitor is used for the manufacture of a medicament for the long-term treatment and administered for at least 10 weeks in a line of treatment. In one aspect of this embodiment, the cMET inhibitor is used for the manufacture of a medicament for the long-term treatment and not administered in combination with chemotherapy in the same line of treatment. In one aspect of this embodiment, the cMET inhibitor is used for the manufacture of a medicament for the long-term treatment and not administered in combination with radiotherapy in the same line of treatment. In one aspect of this embodiment, the cMET inhibitor is used for the manufacture of a medicament for the long-term treatment of a human.

### DETAILED DESCRIPTION

Compounds for use according to the invention advantageously target c-Met to suppress glioblastoma. cMET inhibitors may be a potential strategy for treating a glioblastoma, e.g., by crossing the blood-brain barrier. The result of administration of a compound of the invention may also be to reduce tumor burden, which in turn will reduce the severity of disease. Whatever the exact mechanism of action for the anti-cancer properties of the cMET inhibitors of the invention is, it is proposed that administration thereof may have one or more clinical benefits, as described further herein. In particular, the application describes and demonstrates cMET monotherapy for treating glioblastoma in a subject.

Treatment of glioblastoma using the methods of this invention include administration of an effective amount of a cMET inhibitor of the invention at any stage of the glioblastoma to prevent or reduce the symptoms associated therewith. Typically, subjects will be administered an effective amount of a cMET inhibitor of the invention after definitive diagnosis and presentation with symptoms consistent with a glioblastoma, and administration will reduce the severity of the tumor and/or prevent progression of the tumor to a more severe state. The clinical benefits upon such administration is described in more detail in the sections below.

### Definitions

"A", "an", and "the" include plural referents unless the context clearly dictates otherwise. Thus, for example, reference to a compound refers to one or more compounds or at least one compound. As such, the terms "a" (or "an"), "one or more", and "at least one" are used interchangeably herein.

"About" when used to modify a numerically defined parameter (e.g., the dose of a cMET inhibitor, or the length of treatment time with a therapy described herein) means that the parameter may vary by as much as 10% below or above the stated numerical value for that parameter.

"Administering" or "administration of" a drug to a patient (and grammatical equivalents of this phrase) refers to direct administration, which may be administration to a patient by a medical professional or may be self-administration, and/or indirect administration, which may be the act of prescribing a drug. E.g., a physician who instructs a patient to self-administer a drug or provides a patient with a prescription for a drug is administering the drug to the patient.

"Biomarker" generally refers to biological molecules, and quantitative and qualitative measurements of the same, that are indicative of a disease state. "Prognostic biomarkers" correlate with disease outcome, independent of therapy. E.g., tumor hypoxia is a negative prognostic marker - the higher the tumor hypoxia, the higher the likelihood that the outcome of the disease will be negative. "Predictive biomarkers" indicate whether a patient is likely to respond positively to a particular therapy. E.g., HER2 profiling is commonly used in breast cancer patients to determine if those patients are likely to respond to Herceptin (trastuzumab, Genentech). "Response biomarkers" provide a measure of the response to a therapy and so provide an indication of whether a therapy is working. E.g., decreasing levels of prostate-specific antigen generally indicate that anti-cancer therapy for a prostate cancer patient is working. When a marker is used as a basis for identifying or selecting a patient for a treatment described herein, the marker can be measured before and/or during treatment, and/or the values obtained are used by a clinician in assessing any of the following: (a) probable or likely suitability of an individual to initially receive treatment(s); (b) probable or likely unsuitability of an individual to initially receive treatment(s); (c) responsiveness to treatment; (d) probable or likely suitability of an individual to continue to receive treatment(s); (e) probable or likely unsuitability of an individual to continue to receive treatment(s); (f) adjusting dosage; (g) predicting likelihood of clinical benefits; or (h) toxicity. As would be well understood by one in the art, measurement of a biomarker in a clinical setting is a clear indication that this parameter was used as a basis for initiating, continuing, adjusting and/or ceasing administration of the treatments described herein.

"Chemotherapy" is a therapy involving a chemotherapeutic agent, which is a chemical compound useful in the treatment of cancer.

"Clinical outcome", "clinical parameter", "clinical response", or "clinical endpoint" refers to any clinical observation or measurement relating to a patient's reaction to a therapy. Non-limiting examples of clinical outcomes include tumor response (TR), overall survival (OS), progression free survival (PFS), disease free survival, time to tumor recurrence (TTR), time to tumor progression (TTP), relative risk (RR), toxicity, or side effect.

"cMET inhibitor" refers to a compound that has a biological effect to inhibit or significantly reduce or down-regulate the expression of the gene encoding for cMET and/or the expression of cMET and/or the biological activity of cMET. In one embodiment, the cMET inhibitor specifically binds the cMET kinase. There are two classes of agents with clinical activity against receptor tyrosine kinases: antibodies, which target the extracellular domain of the receptor, and small molecule TKIs, which target the intracellular part by competing with ATP, thus inhibiting the autophosphorylation of the receptor and preventing downstream signaling. Examples of cMET TKIs include tepotinib, crizotinib, capmatinib, savolitinib, cabozantinib, MGCD265, merestinib, and 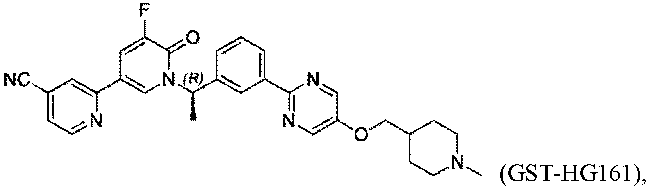 whereas onartuzumab and ARGX-111 are examples of cMET-targeting antibodies.

"Combination" refers to the provision of a first active modality in addition to another active modality. Contemplated with the scope of the combinations described herein, are any regimen of combination modalities or partners (e.g., active compounds, components, or agents), encompassed in single or multiple compositions. It is understood that any modalities within a single composition, formulation, or unit dosage form (i.e., a fixed-dose combination) must have the identical dose regimen and route of delivery. It is not intended to imply that the modalities must be formulated for delivery together (e.g., in the same composition, formulation or unit dosage form). The combined modalities can be manufactured and/or formulated by the same or different manufacturers. The combination partners may thus be, e.g., entirely separate pharmaceutical dosage forms or pharmaceutical compositions that are also sold independently of each other.

"Combination therapy", "in combination with", or "in conjunction with" denotes any form of concurrent, parallel, simultaneous, sequential, or intermittent treatment with at least two distinct treatment modalities (e.g., compounds, components, or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations, or unit dosage forms) or separately (e.g., on the same day or on different days and in any order as according to an appropriate dosing protocol for the separate compositions, formulations, or unit dosage forms) in a manner and dosing regimen prescribed by a medical care taker or according to a regulatory agency. In general, each treatment modality will be administered at a dose and/or on a time schedule determined for that treatment modality. Optionally, three or more modalities may be used in a combination therapy. Additionally, combination therapies may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

"Complete response" or "complete remission" refers to the disappearance of all signs of cancer, including glioblastoma, in response to treatment. This does not always mean the glioblastoma has been cured.

"Comprising" is intended to mean that the compositions and methods include the recited elements, but not excluding others. "Consisting essentially of", when used to define compositions and methods, shall mean excluding other elements of any essential significance to the composition or method. "Consisting of" shall mean excluding more than trace elements of other ingredients for claimed compositions and substantial method steps. Embodiments defined by each of these transition terms are within the scope of this invention. Accordingly, it is intended that the methods and compositions can include additional steps and components (comprising) or alternatively including steps and compositions of no significance (consisting essentially of) or alternatively, intending only the stated method steps or compositions (consisting of).

"Dose" and "dosage" refer to a specific amount of active or therapeutic agents, including cMET inhibitors, for administration. Such amounts are included in a "dosage form," which refers to physically discrete units suitable as unitary dosages for human subjects and other mammals, each unit containing a predetermined quantity of active agent calculated to produce the desired onset, tolerability, and therapeutic effects, in association with one or more suitable pharmaceutical excipients such as carriers.

"Medicament" in the meaning of the invention is any agent in the field of medicine, which comprises one or more compounds of the invention or preparations thereof (e.g., a pharmaceutical composition or pharmaceutical formulation) and can be used in prophylaxis, therapy, follow-up or aftercare of patients who suffer from clinical symptoms and/or known risk to glioblastoma.

"Objective response" refers to a measurable response, including complete response (CR) or partial response (PR).

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period, such as one year, five years, etc., e.g., from the time of diagnosis or treatment.

"Partial response" refers to a decrease in the size of one or more tumors or lesions, or in the extent of cancer in the body, in response to treatment, as defined in the RECIST v1.1 guideline.

"Patient" or "subject" means an animal, preferably a human. However, "subject" can include companion animals such as dogs and cats. In one embodiment, the subject is an adult human patient. In another embodiment, the subject is a pediatric patient. Pediatric patients include any human which is under the age of 18 at the start of treatment. Adult patients include any human which is age 18 and above at the start of treatment. In one embodiment, the subject is a member of a high-risk group, such as being over 65 years of age, harboring MET amplification, or humans with other co-morbidities.

"Pharmaceutically acceptable" indicates that the compound, inhibitor, substance, or composition must be compatible chemically and/or toxicologically, with the other ingredients comprised in a formulation, and/or the subject being treated therewith. In other words, the substance or composition must be chemically and/or toxicologically suitable for the treatment of subjects.

"Pharmaceutically acceptable carrier" or "pharmaceutically acceptable diluent" means all solvents, dispersion media, coatings, antibacterial and antifungal agents, isotonic and absorption delaying agents, compatible with pharmaceutical administration. Examples of pharmaceutically acceptable carriers include one or more of water, saline, phosphate buffered saline, dextrose, glycerol, ethanol, and the like, as well as combinations thereof. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), and polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers may also be included in a pharmaceutical composition described herein, if they do not adversely affect the desired characteristics of the pharmaceutical composition.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation, wherein a patient remains alive, without the cancer, including a glioblastoma, getting worse.

"RECIST" means Response Evaluation Criteria in Solid Tumors. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline, and it is published in 2009, European Journal of Cancers 45: 228.

"Recurrent" cancer, including "recurrent" glioblastoma is one which has re-grown, either at the initial site or at a distant site, after a response to initial therapy, such as surgery. A locally "recurrent" cancer is cancer that returns after treatment in the same place as a previously treated cancer.

"Reduction" of a symptom or symptoms (and grammatical equivalents of this phrase) refers to decreasing the severity or frequency of the symptom(s), or elimination of the symptom(s).

"Resistant" tumors or cancers are tumors or cancers, including glioblastoma, which cannot, or can no longer, be treated with anti-cancer agents. Resistance may already be present before the first treatment attempt with an anti-cancer agent. Resistance may also be acquired after the initial treatment with an anti-cancer agent, in some instances as a result of the treatment.

"Suitable for therapy" or "suitable for treatment" shall mean that the patient is likely to exhibit one or more desirable clinical outcomes as compared to patients having the same cancer and receiving the same therapy but possessing a different characteristic that is under consideration for the purpose of the comparison. In one aspect, the characteristic under consideration is a genetic polymorphism or a somatic mutation. In another aspect, the characteristic under consideration is the expression level of a gene or a polypeptide. In one aspect, a more desirable clinical outcome is relatively higher likelihood of or relatively better tumor response such as tumor load reduction. In another aspect, a more desirable clinical outcome is relatively longer overall survival. In yet another aspect, a more desirable clinical outcome is relatively longer progression free survival or time to tumor progression. In yet another aspect, a more desirable clinical outcome is relatively longer disease-free survival. In another aspect, a more desirable clinical outcome is relative reduction or delay in tumor recurrence. In another aspect, a more desirable clinical outcome is relatively decreased metastasis. In another aspect, a more desirable clinical outcome is relatively lower relative risk. In yet another aspect, a more desirable clinical outcome is relatively reduced toxicity or side effects. In some embodiments, more than one clinical outcome is considered simultaneously. In one such aspect, a patient possessing a characteristic, such as a genotype of a genetic polymorphism, may exhibit more than one more desirable clinical outcomes as compared to patients having the same cancer and receiving the same therapy but not possessing the characteristic. As defined herein, the patient is considered suitable for the therapy. In another such aspect, a patient possessing a characteristic may exhibit one or more desirable clinical outcomes but simultaneously exhibit one or more less desirable clinical outcomes. The clinical outcomes will then be considered collectively, and a decision as to whether the patient is suitable for the therapy will be made accordingly, considering the patient's specific situation and the relevance of the clinical outcomes. In some embodiments, progression free survival or overall survival is weighted more heavily than tumor response in a collective decision making.

"Therapeutically effective amount" of a cMET inhibitor refers to an amount effective, at dosages and for periods of time necessary, whereby, when administered to a patient with a cancer, including a glioblastoma, according to a method, combination or combination therapy, the method, combination or combination therapy, will have the intended therapeutic effect, e.g., alleviation, amelioration, palliation, or elimination of one or more manifestations of the cancer in the patient, or any other clinical result in the course of treating a cancer patient. A therapeutic effect does not necessarily occur by administration of one dose, and it may occur only after administration of a series of doses. Thus, a therapeutically effective amount may be administered in one or more administrations. Such therapeutically effective amount may vary according to factors such as the disease state, age, sex, and weight of the individual, and the ability of a cMET inhibitor, to elicit a desired response in the individual. A therapeutically effective amount is also one in which any toxic or detrimental effects of a cMET inhibitor is outweighed by the therapeutically beneficial effects.

"Treatment," "treat," and "treating" refer to reversing, alleviating, delaying the onset of, or inhibiting the progress of a glioblastoma, or one or more symptoms thereof, as described herein. In some embodiments, treatment is administered after one or more symptoms have developed. In other embodiments, treatment is administered in the absence of symptoms. For example, treatment is administered to a susceptible individual prior to the onset of symptoms (e.g., in light of co-morbidities which are predictors for severe disease, or other susceptibility factors).

"Tumor" as it applies to a subject diagnosed with, or suspected of having, a cancer refers to a malignant or potentially malignant neoplasm or tissue mass of any size, and it includes primary tumors and secondary neoplasms. A solid tumor is an abnormal growth or mass of tissue that usually does not contain cysts or liquid areas. Different types of solid tumors are named for the type of cells that form them.

### Compounds

One embodiment is use of a compound according to the following formula: 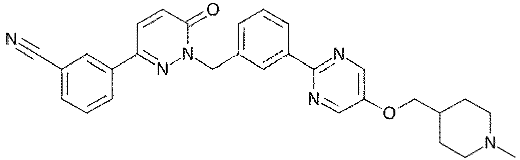 or a pharmaceutically acceptable salt and/or solvate or hydrate thereof for the treatment of a glioblastoma.

The compound may also be referred to as first compound. The first compound may also be referred to as 3-(1-{3-[5-(1-methyl-piperidin-4-ylmethoxy)-pyrimidin-2-yl]-benzyl}-6-oxo-1,6-dihydro-pyridazin-3-yl)-benzonitrile (INN: tepotinib). It is disclosed and further characterized as compound "A257" in WO 2009/006959. In an exemplary embodiment, a hydrochloride hydrate form of this first compound is used, which is referred to as 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate. In another exemplary embodiment, a hydrochloride monohydrate form of this first compound is used, which is referred to as 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate. It is disclosed and further characterized as compound "A7" in WO 2009/007074. In an exemplary embodiment, the crystalline form H2 of the hydrochloride monohydrate of this first compound is used, which is referred to as crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate. It is disclosed and further characterized in Example 12 in WO 2010/078897.

Any reference to the first compound in the following shall be read as including a reference to 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate, 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate, and crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate. In a preferred embodiment, the cMET inhibitor for use in the method of the invention is selected from the group of 3-(1-{3-[5-(1-methyl-piperidin-4-ylmethoxy)-pyrimidin-2-yl]-benzyl}-6-oxo-1,6-dihydro-pyridazin-3-yl)-benzonitrile, 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate, 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate, and crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate. All the compounds described above are highly selective and potent cMET inhibitors.

One embodiment is a compound according to the following formula (GST-HG161): 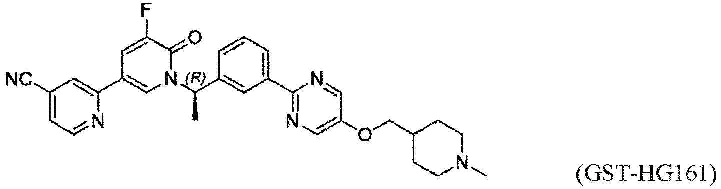 or a pharmaceutically acceptable salt and/or solvate or hydrate thereof for use in the treatment of a glioblastoma. The cMET inhibitor GST-HG161 is disclosed and further characterized as "embodiment 1-2" in EP 3 533 787.

The above compounds may either be used in their free forms or as pharmaceutically acceptable salts. The free compounds may be converted into the associated acid-addition salt by reaction with an acid, for example by reaction of equivalent amounts of the base and the acid in an inert solvent, such as, for example, ethanol, and subsequent evaporation. Suitable acids for this reaction are, in particular, those which give physiologically acceptable salts, such as, for example, hydrogen halides (for example hydrogen chloride, hydrogen bromide or hydrogen iodide), other mineral acids and corresponding salts thereof (for example sulfate, nitrate or phosphate and the like), alkyl- and monoaryl sulfonates (for example ethanedisulfonate (edisylate), toluene sulfonate, napthalene-2-sulfonate (napsylate), benzenesulfonate) and other organic acids and corresponding salts thereof (for example fumarate, oxalate, acetate, trifluoroacetate, tartrate, maleate, succinate, citrate, benzoate, salicylate, ascorbate, and the like.

An exemplary embodiment of pharmaceutically acceptable salts of the first compound comprises the hydrochloride salt, as described above.

Unless otherwise stated, structures depicted herein are also meant to include all isomeric (e.g., enantiomeric, diastereomeric, and geometric (or conformational) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention.

Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. In some embodiments, the group comprises one or more deuterium atoms.

In particular, the cMET inhibitor is not administered in combination with chemotherapy in the same line of treatment. In one embodiment, administration of the cMET inhibitor is excluded in combination with any other therapeutic agent, particularly in combination with another chemotherapeutic agent, in any line of treatment, e.g., prior to, concurrently, or after administration of the cMET inhibitor. In one embodiment, the administration of the cMET inhibitor is excluded in combination with a p70S6k inhibitor, particularly in combination with 4-[(S)-2-azetidin-1-yl-1-(4-chloro-3-trifluoromethyl-phenyl)-ethylamino]-quinazoline-8-carboxylic acid amide. In one embodiment, the cMET inhibitor is not administered in combination therapy. In one embodiment, the cMET inhibitor is administered in combination with radiotherapy, particularly in the same line of treatment.

### Uses, Formulation and Administration

Compositions of the present invention are administered orally, parenterally, by inhalation spray, topically, rectally, nasally, buccally, vaginally or via an implanted reservoir. Preferably, the compositions are administered orally. More preferably, the cMET inhibitor is administered via oral administration. In one embodiment, the oral formulation of a compound of the invention is a tablet or capsule form. In another embodiment, the oral formulation is a solution or suspension which may be given to a subject in need thereof via mouth or nasogastric tube. Any oral formulations of the invention may be administered with or without food. In some embodiments, pharmaceutically acceptable compositions of this invention are administered without food. In other embodiments, pharmaceutically acceptable compositions of this invention are administered with food.

Pharmaceutically acceptable compositions of this invention are orally administered in any orally acceptable dosage form. Exemplary oral dosage forms are capsules, tablets, aqueous suspensions, or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring, or coloring agents are optionally also added.

The amount of compound of the present invention that are optionally combined with the carrier materials to produce a composition in a single dosage form will vary depending upon the host treated, and the particular mode of administration. Preferably, provided compositions should be formulated so that a dosage of between 0.01 - 100 mg/kg body weight/day of the compound can be administered to a patient receiving these compositions.

The pharmaceutical composition is in the form of one or more dosage units, which is provided either as a single dose per day or in a series of doses of two or more per day so that the total daily dose would be the same as for the single dose per day. In certain embodiments, the cMET inhibitor is administered in a dosage strength of about 15 mg, 25 mg, 100 mg, 215 mg, 225 mg, 250 mg, 300 mg, 450 mg, 500 mg or 1000 mg, preferably about 15 mg, 25 mg, 100 mg, 225 mg, 250 mg, 450 mg, or 500 mg, more preferably about 225 mg or 250 mg.

In certain embodiments, the total amount of cMET inhibitor administered to the subject in need thereof is between about 0.5 mg to about 1400 mg per day, preferably between about 300 mg to about 1400 mg per day, more preferably between about 225 mg and about 1000 mg per day. In certain embodiments, the total amount of cMET inhibitor administered to the subject in need thereof is between about 1 mg and 1000 mg per day, more preferably between about 1 mg and 700 mg per day, most preferably between about 100 mg and 500 mg per day, highly preferably between about 225 mg and 500 mg per day. In one embodiment, the total amount of cMET inhibitor administered to the subject in need thereof is between about 225 mg and 900 mg per day, preferably 225 mg and 450 mg per day, more preferably 450 mg. In one embodiment, the total amount of cMET inhibitor administered to the subject in need thereof is about 900 mg per day. In one embodiment, the total amount of cMET inhibitor administered to the subject in need thereof is between about 250 mg and 1000 mg per day, preferably 250 mg and 500 mg per day, more preferably 500 mg. In one embodiment, the total amount of cMET inhibitor administered to the subject in need thereof is about 1000 mg per day. In certain embodiments, the total amount of cMET inhibitor administered to the subject in need thereof is between about 30 mg and 400 mg per day, more preferably between about 30 mg to 230 mg per day. In one embodiment, the total amount of cMET inhibitor administered to the subject in need thereof is between about 60 mg to 315 mg per day.

In any of the above embodiments, the cMET inhibitor is administered daily. In any of the above embodiments, the cMET inhibitor is administered once a day. In one embodiment, the cMET inhibitor is administered in a dosage strength of about 450 mg to 500 mg once a day. In one embodiment, the dosage of tepotinib is 450 mg (equivalent to 500 mg tepotinib hydrochloride hydrate) orally once daily. In one embodiment, the dosage of tepotinib is 225 mg orally once daily. 225 mg tepotinib is equivalent to 250 mg tepotinib hydrochloride hydrate. In one embodiment, the dosage of tepotinib is 900 mg orally once daily. 900 mg tepotinib is equivalent to 1000 mg tepotinib hydrochloride hydrate.

In any of the above embodiments, the cMET inhibitor is administered twice a day. In one embodiment, the cMET inhibitor is administered in a dosage strength of 250 mg twice a day. In one embodiment, the cMET inhibitor is administered in a dosage strength of 500 mg twice a day. In one embodiment, the dosage of tepotinib is 450 mg orally twice daily.

In any of the above embodiments, the cMET inhibitor is administered three times a day. In one embodiment, the cMET inhibitor is administered in a dosage strength of about 100 mg three times a day.

In any of the above embodiments, the cMET inhibitor is administered four times a day. In one embodiment, the cMET inhibitor is administered in a dosage strength of 250 mg four times a day. In one embodiment, the dosage of tepotinib is 225 mg orally four times daily.

In any of the above embodiments, the cMET inhibitor is administered three times a week. In one embodiment, the cMET inhibitor is administered in a dosage strength between about 60 mg and 315 mg three times a week.

In any of the above embodiments, the cMET inhibitor is administered for a period of at least 10 weeks. In one aspect of any of the above embodiments, the cMET inhibitor is administered for a period of at least about 11 weeks, 12 weeks, 13 weeks, 14, weeks, 15 weeks, 16 weeks, 17 weeks, 18 weeks, 19, weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 41 weeks, 42 weeks, 43 weeks, 44 weeks, 45 weeks, 46 weeks, 47 weeks, 48 weeks, 49 weeks, 50 weeks, 51 weeks, or 52 weeks. In one aspect of any of the above embodiments, the cMET inhibitor is administered for a period of at least about 11 weeks to about 52 weeks, preferably at least about 11-25 weeks, more preferably at least about 15-21 weeks, most preferably at least about 18-20 weeks, highly preferably at least about 19 weeks.

In another aspect of any of the above embodiments, the cMET inhibitor is administered for at least about 10-52 weeks, particularly for at least about 10-19 weeks. In one embodiment, the cMET inhibitor is administered in a dosage strength of about 300 mg to 1400 mg once a day for at least about 10-52 weeks, particularly for at least about 10-19 weeks. In one embodiment, the cMET inhibitor is administered in a dosage strength of about 450 mg to 500 mg twice daily for at least about 10-52 weeks, particularly for at least about 10-19 weeks. In one embodiment, tepotinib hydrochloride hydrate is administered in a dosage strength of about 500 mg twice daily for at least about 10-52 weeks, particularly for at least about 10-19 weeks.

In one embodiment of the invention, the subject is suffering from a disseminated glioblastoma. In one embodiment of the invention, a disseminated glioblastoma is treated. In one embodiment of this invention, the subject is suffering from a glioblastoma harboring MET amplification, particularly at 7q31.2. In one embodiment of the invention, a glioblastoma harboring MET amplification is treated. In one embodiment of the invention, the subject is suffering from a disseminated glioblastoma and/or a glioblastoma harboring MET amplification. In one embodiment of the invention, a disseminated glioblastoma, a glioblastoma harboring MET amplification, or a combination thereof, is treated. MET amplification is caused by an increase in the copy number of the MET gene and has been identified as a resistance mechanism in EGFR mutation-positive NSCLC (Socinski et al., 2021, JCO Precision Oncology 5: 653). In one embodiment of this invention, the subject is suffering from a glioblastoma harboring IDH wild-type, MGMT-unmethylated and/or MET amplification at 7q31.2. In one embodiment of this invention, a glioblastoma harboring IDH wild-type, MGMT-unmethylated or MET amplification at 7q31.2, or a combination thereof, is treated. In another aspect of this embodiment, the glioblastoma harbors MET exon 14 skipping.

These biomarkers can be determined by standard methods. In various embodiments, the biomarker protein level is determined by a method comprising quantitative western blot, multiple immunoassay formats, ELISA, immunohistochemistry, histochemistry, or use of FACS analysis of tumor lysates, immunofluorescence staining, a bead-based suspension immunoassay, Luminex technology, or a proximity ligation assay. In another embodiment, the biomarker RNA level is determined by a method comprising microarray chips, RT-PCR, qRT-PCR, multiplex qPCR, or in-situ hybridization. In another embodiment, the marker is detected by next generation sequencing, liquid biopsy and/or tissue biopsy. For example, MET exon 14 skipping can be detected by DNA-based methods, e.g., next-generation sequencing or Sanger sequencing, as well as RNA-based methods, e.g., next-generation sequencing or quantitative PCR assays, in each case using samples from liquid biopsy (e.g., using the Guardant360 CDx Test Version 2.10 (73 genes) assay) or tissue biopsy.

Any suitable sample can be used for the method. Non-limiting examples of such include one or more of a serum sample, plasma sample, whole blood, pancreatic juice sample, tissue sample, tumor lysate or a tumor sample, which can be isolated, e.g., from a needle biopsy, core biopsy, or needle aspirate. For example, tissue, plasma, or serum samples are taken from the patient before treatment and optionally on treatment with the cMET inhibitor of the invention. The information obtained may indicate whether a patient will respond favorably or unfavorably to cancer therapy.

In one embodiment of the invention, the subject is being treated inpatient in a hospital setting. In another embodiment, the subject is being treated in an outpatient setting. In one aspect of the preceding embodiments, the subject may continue administration of the cMET inhibitor after being transitioned from being treated from an inpatient hospital setting to an outpatient setting.

In one embodiment, the administration of the cMET inhibitor results in one or more clinical benefits. In one aspect of this embodiment, the one or more clinical benefits are selected from the group comprising: complete response, increase in the likelihood of complete response, reduction in the likelihood of development of drug resistance, extension in time to tumor progression, and extension in time to tumor recurrence in the subject; preferably complete response.

The invention also provides a method of treating a glioblastoma in a subject in need thereof comprising administering an effective amount of a compound of the invention to the subject. An amount effective to treat or inhibit a glioblastoma is an amount that will cause a reduction in one or more of the manifestations of glioblastoma, such as tumor burden and mortality as compared to untreated control subjects.

One embodiment of the invention is a method of treating a glioblastoma in a subject in need thereof, comprising administering an effective amount of a cMET inhibitor, or a pharmaceutically acceptable salt and/or solvate or hydrate thereof to the subject. In one aspect of this embodiment, the subject is human. In one aspect of this embodiment, the cMET inhibitor is administered to the subject for at least 10 weeks. In one aspect of this embodiment, the cMET inhibitor is tepotinib or a pharmaceutically acceptable salt, solvate and/or hydrate thereof. In one aspect of this embodiment, the cMET inhibitor is not administered in combination with 4-[(S)-2-azetidin-1-yl-1-(4-chloro-3-trifluoromethyl-phenyl)-ethylamino]-quinazoline-8-carboxylic acid amide. In one aspect of this embodiment, the cMET inhibitor is administered for at least 10 weeks in a single line of treatment. In one aspect of this embodiment, the cMET inhibitor is not administered in combination with chemotherapy in the same line of treatment.

One preferred embodiment of the invention is a method for treating a glioblastoma in a human in need thereof, comprising administering an effective amount of tepotinib, or a pharmaceutically acceptable salt, solvate and/or hydrate thereof, to the human for at least 10 weeks, wherein tepotinib is not administered in combination with chemotherapy in a line of treatment. More specifically, one preferred embodiment of the invention is a method for long-term treatment of a glioblastoma in a human subject in need thereof, comprising administering an effective amount of a cMET inhibitor to the subject for at least 10 weeks in a line of treatment, wherein the cMET inhibitor is 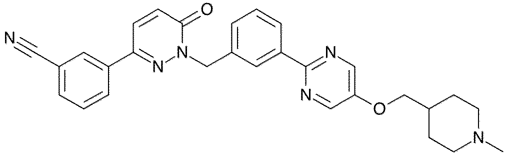 or a pharmaceutically acceptable salt, solvate and/or hydrate thereof, and wherein the cMET inhibitor is not administered in combination with chemotherapy in the same line of treatment.

In another aspect of this embodiment, the administration of the cMET inhibitor results in anti-tumor activity in the subject in the long term.

The terms "same line of treatment", "one and the same line of treatment", "specific line of treatment", and "single line of treatment" are interchangeably used herein and refer to a defined line of treatment, i.e., first-line, second-line, or higher line of treatment, but not a combination thereof, unless context indicates otherwise.

In various embodiments, the method of the invention is employed as a first, second, third, or later line of treatment. A line of treatment refers to a place in the order of treatment with different medications or other therapies received by a patient. First-line therapy regimens are treatments given first, whereas second- or third-line therapy is given after the first-line therapy or after the second-line therapy, respectively. Therefore, first-line therapy is the first treatment for a disease or condition. In patients with cancer, including glioblastoma, first-line therapy, sometimes referred to as primary therapy or primary treatment, can be surgery, chemotherapy, radiation therapy, or a combination of these therapies. Typically, a patient is given a subsequent chemotherapy regimen (second- or third-line therapy), either because the patient did not show a positive clinical outcome or only showed a sub-clinical response to a first- or second-line therapy or showed a positive clinical response but later experienced a relapse, sometimes with disease now resistant to the earlier therapy that elicited the earlier positive response.

In another aspect of this embodiment, the cMET inhibitor is administered in a first-line treatment of the glioblastoma. In one aspect of this embodiment, the cMET inhibitor is administered in a first-line treatment of the glioblastoma, and the method further comprises administering a treatment, or line of treatment, selected from the group of surgery, radiotherapy, chemotherapy, and a combination thereof, after first administration of the cMET inhibitor. The safety and the clinical benefit offered by the cMET inhibitor of the invention warrants first-line setting in glioblastoma patients. Particularly, tepotinib may become a new standard treatment for patients suffering from a glioblastoma. In another aspect of this embodiment, the cMET inhibitor is administered in a second-line treatment or higher line of treatment of the glioblastoma.

In another aspect of this embodiment, the cMET inhibitor is administered in a second-line treatment or higher line of treatment of the glioblastoma, and the method further comprises administering a treatment, or line of treatment, selected from the group of surgery, radiotherapy, chemotherapy, and a combination thereof, prior to first administration of the cMET inhibitor. There is no limitation to the prior number of therapies provided that the subject underwent at least one round of prior cancer therapy. The round of prior cancer therapy refers to a defined schedule/phase for treating a subject with, e.g., chemotherapeutic agents (excluding cMET inhibitors according to the invention), radiotherapy or chemoradiotherapy, and the subject failed with such previous treatment, which was either completed or terminated ahead of schedule. One reason could be that the cancer was resistant or became resistant to prior therapy. The use of the cMET inhibitor will suppress this mechanism of resistance and restore the effect of therapy. The set of patients with resistance becomes treatable and shows improved responses. In one aspect, the subject underwent at least one round or line of prior glioblastoma treatment; wherein, optionally, the glioblastoma was resistant or became resistant to prior treatment. Round of prior glioblastoma therapy and line of prior glioblastoma treatment are interchangeably used herein. In one aspect of this embodiment, the subject has been previously treated with surgery, radiotherapy and/or chemotherapy.

In any of the above embodiments, the administration of the cMET inhibitor results in one or more clinical benefits to the subject. In one aspect of this embodiment, the one or more clinical benefits are complete response, increase in the likelihood of complete response, reduction in the likelihood of development of drug resistance, extension in time to tumor progression, and extension in time to tumor recurrence.

The compounds of the invention can be administered before or following an onset of a glioblastoma, or after glioblastoma has been diagnosed in a subject. The aforementioned compounds and medical products of the inventive use are particularly used for the therapeutic treatment. A therapeutically relevant effect relieves to some extent one or more symptoms of a disorder, or returns to normality, either partially or completely, one or more physiological or biochemical parameters associated with or causative of a disease or pathological condition. Monitoring is considered as a kind of treatment provided that the compounds are administered in distinct intervals, e.g., to boost the response and eradicate the symptoms of the disease. The methods of the invention can also be used to reduce the likelihood of developing a disorder or even prevent the initiation of disorders associated with glioblastoma in advance of the manifestation of mild to moderate disease, or to treat the arising and continuing symptoms of a glioblastoma.

The invention furthermore relates to a medicament comprising at least one compound for use according to the invention or a pharmaceutically acceptable salt, solvate and/or hydrate thereof.

### Monotherapy

The invention relates to the successful long-term therapy of glioblastoma in a subject. In various embodiments, the cMET inhibitor is administered alone, i.e., as monotherapy. In one embodiment, the cMET inhibitor is administered as monotherapy in a specific line of treatment. It does not exclude the administration of other treatments in another line of treatment before or after the monotherapy with the cMET inhibitor. In one embodiment, the cMET inhibitor is administered as monotherapy overall. It is a single active-ingredient treatment and the only line of treatment (i.e., first-line treatment).

In various embodiments, the cMET inhibitor is not administered in combination with 4-[(S)-2-azetidin-1-yl-1-(4-chloro-3-trifluoromethyl-phenyl)-ethylamino]-quinazoline-8-carboxylic acid amide. Preferably, the cMET inhibitor is administered without any p70S6k inhibitor, more preferably without any chemotherapeutic agent, most preferably without any chemotherapy or radiotherapy, highly preferably without any other therapeutic agent (hereinafter jointly referred to as limited combination therapy).

It has been surprisingly found by the inventors that long-term treatment without resistance is successful if the cMET inhibitor is administered as monotherapy (or limited combination therapy) for at least 10 weeks or more, preferably up to 19 weeks or more, more preferably up to 52 weeks or more. Prior art does not teach or suggest that a cMET inhibitor, particularly tepotinib, can be used to treat glioblastoma successfully as a monotherapy (or limited combination therapy) without development of drug resistance and tumor progression.

### Combination treatment

In various embodiments, the cMET inhibitor is administered in combination with surgery, radiotherapy, chemotherapy, and a combination thereof. They could be administered simultaneously, separately, or sequentially and in any order. The individual combination partners of a combination therapy of the invention may be administered separately at different times during therapy or concurrently in divided or single combination forms. The treatment regimens in the combination may have different but overlapping delivery regimens, e.g., daily, twice daily, vs. a single or weekly administration. In one aspect of these embodiments, the method further comprises comprising administering a treatment, or line of treatment, selected from the group of surgery, radiotherapy, chemotherapy, and a combination thereof, prior to first administration of the cMET inhibitor. Optionally, the surgery or radiotherapy, or the combination thereof, is administered first, followed by chemotherapy, prior to the first administration of the cMET inhibitor. In one aspect of these embodiments, the method further comprises administering a treatment selected from the group of surgery radiotherapy, and a combination thereof, optionally followed by chemotherapy, prior to first administration of the cMET inhibitor.

In one embodiment, the cMET inhibitor is administered in combination with surgery. In one aspect of this embodiment, the surgery is lobectomy and/or resection.

In one embodiment, the cMET inhibitor is administered in combination with radiotherapy. The cMET inhibitor and radiotherapy can be administered in any line of treatment, either in one and the same line of treatment or in different lines of treatment. In one aspect of these embodiments, the radiotherapy is administered in a line of prior glioblastoma treatment, i.e., prior to first administration of the cMET inhibitor in a higher line of treatment of the glioblastoma. In one aspect of these embodiments, the cMET inhibitor and radiotherapy are administered in the same line of treatment. In a preferred embodiment of the same-line treatment, the radiotherapy is administered as single irradiation dose or after first administration of the cMET inhibitor, or a combination thereof. In a more preferred embodiment of the same-line treatment, the radiotherapy is administered within 96 hours after first administration of the cMET inhibitor, preferably within 72 hours, more preferably within 48 hours, most preferably within 36 hours, highly preferably within 24 hours. In one embodiment, the radiotherapy is administered within 36 hours after first administration of the cMET inhibitor in the same line of treatment.

Examples of radiotherapy are proton therapy, gamma-radiation, neutron beam radiotherapy, electron beam radiotherapy, brachytherapy, low-dose radiotherapy, systemic radioactive isotopes, and intensity-modulated radiotherapy (IMRT). In one embodiment, the radiotherapy is proton irradiation.

In some embodiments, the radiotherapy is given either with standard fractionation (e.g., 1.8 to 2 Gy a day for 5 days a week) up to a total dose of 50-70 Gy in once daily. Other fractionation schedules could also be envisioned, for example, a lower dose per fraction but given twice daily with the cMET inhibitor given also twice daily. Higher daily doses over a shorter period can also be given. In one embodiment, the radiotherapy comprises 1000-6000 cGy, preferably 2000-5000 cGy, more preferably 3000-4000 cGy, most preferably 3500-3700 cGy. In one aspect of this embodiment, the radiotherapy is followed by boost to the tumor bed at 1000-4000 cGy, preferably 2000-3000 cGy, more preferably 2300-2500 cGy. In a preferred embodiment, the radiotherapy, such as proton radiotherapy, comprises about 3000-4000 cGy, optionally followed by boost to tumor bed at 2000-3000 cGy.

In one embodiment, the cMET inhibitor is administered in combination with one or more additional therapeutic agents with the proviso that the cMET inhibitor is not administered in combination with a chemotherapeutic agent in the same line of treatment. A synergistic or augmented effect may be achieved by using more than one compound in the pharmaceutical composition. The active ingredients can be used either simultaneously or sequentially. In one embodiment, the cMET inhibitor is administered in combination with an anti-cancer agent with the proviso that the cMET inhibitor and the anti-cancer agent are administered in different lines of treatments. In one aspect of this embodiment, the cMET inhibitor is administered in a second-line treatment or higher line of treatment of the glioblastoma, and the chemotherapy is administered prior to first administration of the cMET inhibitor. In one aspect of this embodiment, the cMET inhibitor is administered in a first-line treatment of the glioblastoma, and the chemotherapy is administered after first administration of the cMET inhibitor. In one aspect of this embodiment, the chemotherapy or anti-cancer agent is temozolomide.

In some embodiments, the combination of a cMET inhibitor with one or more additional therapeutic agents reduces the effective amount (including, but not limited to, dosage volume, dosage concentration, and/or total drug dose administered) of the cMET inhibitor and/or the one or more additional therapeutic agents administered to achieve the same result as compared to the effective amount administered when the cMET inhibitor or the additional therapeutic agent is administered alone. In some embodiments, the combination of a cMET inhibitor with the additional therapeutic agent reduces the total duration of treatment compared to administration of the additional therapeutic agent alone. In some embodiments, the combination of a cMET inhibitor with the additional therapeutic agent reduces the side effects associated with administration of the additional therapeutic agent alone. In some embodiments, the combination of an effective amount of the cMET inhibitor with the additional therapeutic agent is more efficacious compared to an effective amount of the cMET inhibitor or the additional therapeutic agent alone. In one embodiment, the combination of an effective amount of the cMET inhibitor with the one or more additional therapeutic agent results in one or more additional clinical benefits than administration of either agent alone.

### Items of the invention

The present invention also comprises the following items:
1. A method for long-term treatment of a glioblastoma in a human subject in need thereof, comprising administering an effective amount of a cMET inhibitor to the subject for at least 10 weeks, wherein the cMET inhibitor is 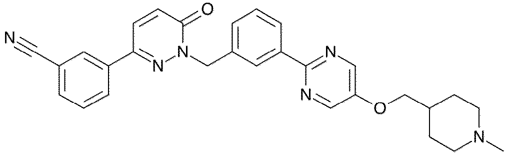 or a pharmaceutically acceptable salt, solvate and/or hydrate thereof, which is not administered in combination with 4-[(S)-2-azetidin-1-yl-1-(4-chloro-3-trifluoromethylphenyl)-ethylamino]-quinazoline-8-carboxylic acid amide.
2. The method of item 1, wherein the cMET inhibitor is selected from the group of 3-(1-{3-[5-(1-methyl-piperidin-4-ylmethoxy)-pyrimidin-2-yl]-benzyl}-6-oxo-1,6-dihydro-pyridazin-3-yl)-benzonitrile, 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate, 3-(1-{3-[5-(1-methyl-piperidin-4-ylmethoxy)-pyrimidin-2-yl]-benzyl} -6-oxo-1,6-dihydro-pyridazin-3-yl)-benzonitrile hydrochloride monohydrate and crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate.
3. The method of any one of the preceding items, wherein disseminated glioblastoma is treated.
4. The method of any one of the preceding items, wherein glioblastoma harboring MET amplification is treated.
5. The method of any one of the preceding items, wherein glioblastoma harboring IDH wild-type, MGMT-unmethylated and/or MET amplification at 7q31.2 is treated.
6. The method of any one of the preceding items, wherein the administration of the cMET inhibitor results in one or more clinical benefits, which are selected from the group of increase in the likelihood of complete response, reduction in the likelihood of development of drug resistance, extension in time to tumor progression, and extension in time to tumor recurrence.
7. The method of any one of the preceding items, wherein the cMET inhibitor is administered for at least about 11-25 weeks.
8. The method of any one of the preceding items, wherein the cMET inhibitor is administered daily.
9. The method of any one of the preceding items, wherein the total amount of cMET inhibitor administered is between about 225 mg and about 1000 mg per day.
10. The method of any one of the preceding items, wherein the cMET inhibitor is administered via oral administration.
11. The method of any one of the preceding items, further comprising administering a treatment selected from the group of surgery, radiotherapy, chemotherapy, and a combination thereof.
12. The method of item 11, wherein the radiotherapy is administered; wherein, optionally, the radiotherapy is proton irradiation.
13. The method of item 11 or 12, wherein the radiotherapy comprises about 3000-4000 cGy, optionally followed by boost to tumor bed at 2000-3000 cGy.
14. The method of item 11, wherein the chemotherapy is administered; wherein, optionally, the chemotherapy is temozolomide.
15. The method of any one of the preceding items, wherein the subject underwent at least one round of prior glioblastoma treatment; wherein, optionally, the glioblastoma was resistant or became resistant to prior treatment.
16. The method of any one of the preceding items, wherein the cMET inhibitor is administered in a second-line treatment or higher line of treatment of the glioblastoma.
17. The method of any one of the preceding items, further comprising administering a treatment selected from the group of surgery, radiotherapy, and a combination thereof, optionally followed by chemotherapy, prior to first administration of the cMET inhibitor.
18. The method of any one of items 1-14, wherein the cMET inhibitor is administered in a first-line treatment of the glioblastoma.
19. The method of any one of items 1-10, wherein the cMET inhibitor is not administered in combination with radiotherapy or chemotherapy.
20. The method of any one of items 1-10, wherein the cMET inhibitor is administered as monotherapy.

It is to be understood that this invention is not limited to the particular compounds, pharmaceutical compositions, uses, and methods described herein, as such matter can, of course, vary. It is also to be understood that the terminology used herein is for the purpose of describing particular embodiments only and is not intended to limit the scope of the present invention, which is only defined by the appended claims. The techniques that are essential according to the invention are described in detail in the specification. Other techniques which are not described in detail correspond to known standard methods that are well known to a person skilled in the art, or the techniques are described in more detail in cited references, patent applications or standard literature. Provided that no other hints in the application are given, they are used as examples only, they are not considered to be essential according to the invention, but they can be replaced by other suitable tools and biological materials.

### EXEMPLIFICATION

As depicted in the Examples below, in certain exemplary embodiments, compounds are prepared according to the following general procedures.

### Example 1: Synthesis

3-(1-{3-[5-(1-methyl-piperidin-4-ylmethoxy)-pyrimidin-2-yl]-benzyl}-6-oxo-1,6-dihydro-pyridazin-3-yl)-benzonitrile (free base), referred to as compound "A257", can be synthesized as described in WO 2009/006959, example 40, and WO 2009/007074, example 3, as follows:

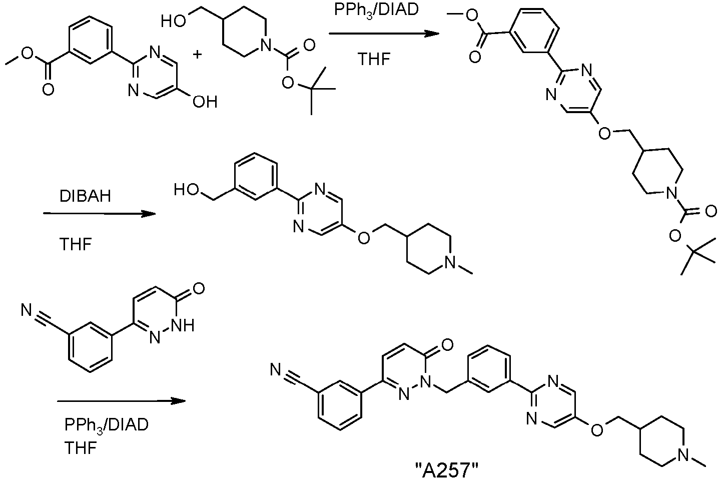

To a suspension of 13.0 g (56.5 mmol) of 3-(5-hydroxy-pyrimidin-2-yl)-benzoic acid methylester and 13.4 g (62.1 mmol) of N-Boc-piperidinemethanol in 115 ml THF 17.7 g (67.8 mmol) of triphenyl-phosphine were given. The suspension was cooled down to 5°C. To the suspension kept at this temperature 13.3 ml (67.8 mmol) of diisopropylazodicarboxylate were given dropwise under stirring within 45 minutes. The reaction mixture was stirred at room temperature for one hour. Subsequently, further 22.2 g (84.7 mmol) of triphenylphosphine and 16.6 ml (84.7 mmol) of diisopropylazodicarboxylate were added. The reaction mixture was stirred at room temperature for 18 hours and concentrated in vacuo. The resulting solid of 4-[2-(3-methoxycarbonyl-phenyl)-pyrimidin-5-yloxymethyl]-piperidine-1-carbonic acid tert.-butylester was sucked off, washed with diethyl ether, and subjected to chromatography (silica gel column and dichloromethane/methanol as eluent/mobile phase).

To a suspension of 1.71 g (3.99 mmol) of 4-[2-(3-methoxycarbonyl-phenyl)-pyrimidin-5-yloxymethyl]-piperidine-1-carbonic acid tert.-butylester in 20 ml THF 25 ml (25 mmol) of a 1 M solution of diisobutylaluminiumhydride in THF were given dropwise under nitrogen. The reaction mixture was stirred for one hour at room temperature and mixed with a saturated solution of sodium sulfate. The resulting precipitate was sucked off and washed with THF and hot 2-propanol. The filtrate was concentrated and re-crystallized from tert.-butylmethylether, resulting in {3-[5-(1-methyl-piperidin-4-ylmethoxy)-pyrimidin-2-yl]-phenyl}-methanol as beige crystals.

To a solution of 313 mg (1.00 mmol) of {3-[5-(1-methyl-piperidin-4-ylmethoxy)-pyrimidin-2-yl]-phenyl}-methanol in 2 ml THF 264 mg (1.30 mmol) of 3-(6-oxo-1,6-dihydro-pyridazin-3-yl)-benzonitrile and 397 mg (1.5 mmol) triphenylphosphine were added subsequently. The reaction mixture was cooled in an ice bath and 294 µl (1.5 mmol) of diisopropylazodicarboxylate are added dropwise. The reaction mixture was stirred at room temperature for 18 hours and then concentrated. The residue was subjected to chromatography (silica gel column and dichloromethane/methanol as eluent/mobile phase). The product containing fractions were pooled, concentrated, and the residue of 3-(1-{3-[5-(1-methyl-piperidin-4-ylmethoxy)-pyrimidin-2-yl]-benzyl}-6-oxo-1,6-dihydro-pyridazin-3-yl)-benzonitrile was decocted with tert.-butylmethylether, sucked off and dried in vacuo.

By means of salt formation, the hemi sulfate, citrate, tartrate, sulfate, succinate, and hydrochloride can be obtained from compound "A257".

Alternatively, 3-(1-{3-[5-(1-methyl-piperidin-4-ylmethoxy)-pyrimidin-2-yl]-benzyl}-6-oxo-1,6-dihydro-pyridazin-3-yl)-benzonitrile (free base) can be synthesized as described in WO 2009/006959, example 43, as follows:

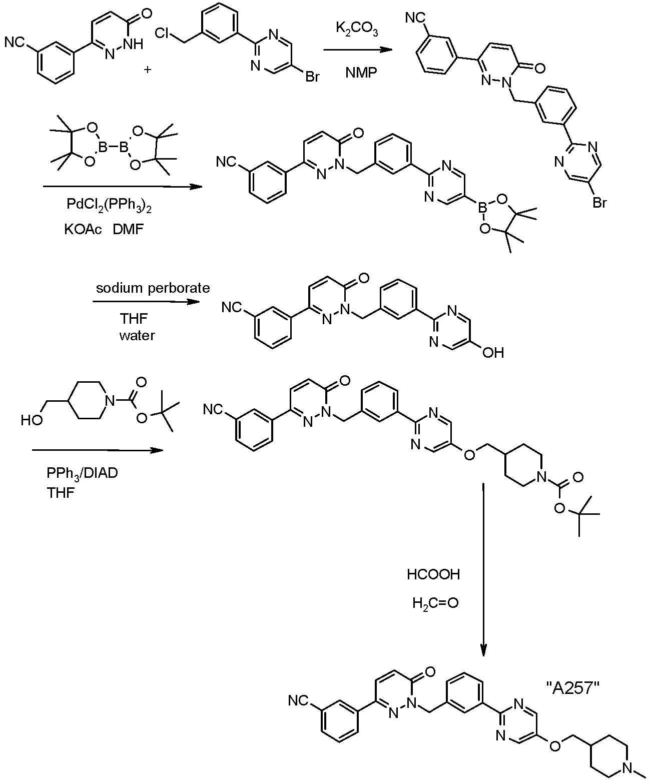

To a suspension of 4.15 g (20 mmol) of 3-(6-oxo-1,6-dihydro-pyridazin-3-yl)-benzonitrile in 40 ml of 1-methyl-2-pyrrolidon 6.00 g (21 mmol) of 5-bromo-2-(3-chloromethyl-phenyl)-pyrimidine and 2.76 g (341 mmol) of potassium carbonate were given. The reaction mixture was stirred at 80°C for 18 hours. Subsequently, the reaction mixture was given onto 200 ml water. The resulting precipitate of 3-{1-[3-(5-bromopyrimidin-2-yl)-benzyl]-6-oxo-1,6-dihydro-pyridazin-3-yl}-benzonitrile was sucked off, washed with water, and dried in vacuo.

To a solution of solution of 18.0 g (41.0 mmol) of 3-{1-[3-(5-bromopyrimidin-2-yl)-benzyl]-6-oxo-1,6-dihydro-pyridazin-3-yl}-benzonitrile in 85 ml DMF 11.8 g (47 mmol) of bis(pinacolato)diboron and 11.9 g (122 mmol) of potassium acetate were given. The reaction mixture was heated up to 80°C under nitrogen. After 15 minutes of stirring at this temperature 273 mg (1.22 mmol) of palladium(II)-acetate were added, and the reaction mixture was stirred for 2 hours at 80°C under nitrogen. Subsequently, the reaction mixture was allowed to cool down to room temperature before the addition of water and dichloromethane, filtration over diatomite/kieselguhr, and separation of the organic phase. The organic phase was dried over sodium sulphate and concentrated yielding 3-(6-oxo-1-{3-[5-(4,4,5,5-tetramethyl-[1,3,2]dioxaborolan-2-yl)-pyrimidin-2-yl]-benzyl}-1,6-dihydro-pyridazin-3-yl)-benzonitrile as grey solid, which could be used for subsequent reactions without purification.

To a suspension of 5.33 g (10.9 mmol) of 3-(6-oxo-1-{3-[5-(4,4,5,5-tetramethyl-[1,3,2]dioxaborolan-2-yl)-pyrimidin-2-yl]-benzyl}-1,6-dihydro-pyridazin-3-yl)-benzonitrile in 35 ml THF and 35 ml water 4.93 g (49.4 mmol) of sodium perborate were given in portions under ice cooling before it is stirred at room temperature for 2 hours. The reaction mixture was mixed with 300 ml of dichloromethane and 100 ml of saturated ammonium chloride solution. The organic phase was separated, dried over sodium sulphate, and concentrated. The residue of 3-{1-[3-(5-hydroxy-pyrimidin-2-yl)-benzyl]-6-oxo-1,6-dihydro-pyridazin-3-yl}-benzonitrile was re-crystallized from methanol.

To a suspension of 25 g (65.6 mmol) of 3-{1-[3-(5-hydroxy-pyrimidin-2-yl)-benzyl]-6-oxo-1,6-dihydro-pyridazin-3-yl}-benzonitrile in 250 ml THF 15.6 g (68.8 mmol) of N-Boc-4-piperidine-methanol and 19.1 g (72.1 mmol) of triphenylphosphine were subsequently added. Then, 14.9 ml (72.1 mmol) of diisopropylazodicarboxylate were added dropwise under ice cooling. The resulting solution was stirred at room temperature for 2 hours. The reaction mixture was further mixed with 750 ml of 2-propanol and 13.1 ml of a 0.5 M solution of potassium hydroxide in ethanol. The resulting precipitate of 4-(2-{3-[3-(3-cyano-phenyl)-6-oxo-6H-pyridazin-1-ylmethyl]-phenyl}-pyrimidin-5-yloxymethyl)-piperidine-1-carbonic acid tert.-butylester was sucked off, washed with diethyl ether, and dried in vacuo.

To a solution of 16.0 g (28.0 mmol) of 4-(2-{3-[3-(3-cyano-phenyl)-6-oxo-6H-pyridazin-1-ylmethyl]-phenyl}-pyrimidin-5-yloxymethyl)-piperidine-1-carbonic acid tert.-butylester in 80 ml formic acid 6.60 ml of 35% aqueous formaldehyde solution were given. The reaction mixture was stirred at a temperature of 110°C for 2 hours before 300 ml water were added. The reaction mixture was concentrated in vacuo to a volume of 150 ml and then extracted with 200 ml of dichloromethane. The organic phase was washed with sodium bicarbonate solution, dried over sodium sulphate, and concentrated. The residue of 3-(1-{3-[5-(1-methyl-piperidin-4-ylmethoxy)-pyrimidin-2-yl]-benzyl}-6-oxo-1,6-dihydro-pyridazin-3-yl)-benzonitrile was re-crystallized from 2-propanol.

The crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate can be prepared as described in WO 2010/078897, example 12, as follows:

Approx. 511 mg of 3-(1-{3-[5-(1-methyl-piperidin-4-ylmethoxy)-pyrimidin-2-yl]-benzyl}-6-oxo-1,6-dihydro-pyridazin-3-yl)-benzonitrile were dispersed in 75 mL acetone, and approx. 1.12 mL of 1 N aqueous HCl solution were added. No clear solution was obtained. However, the remaining solid-state residue was removed by filtration to yield a clear solution afterwards. The resulting clear solution was then incubated overnight, whereupon crystals were obtained. The crystals were separated by filtration and dried for 1 h in a vacuum drying cabinet at 65 °C (purification option 1) or the crystals were separated by filtration, washed with acetone, and dried in a vacuum drying cabinet (purification option 2).

A Powder X-Ray Diffraction pattern of compound "A7" was obtained by standard techniques as described in the European Pharmacopeia 6th Edition chapter 2.9.33 (Cu-Kα1 radiation, λ = 1.5406 Å, Stoe StadiP 611 KL diffractometer). Compound "A7" is characterized by the following XRD data:
Powder X-ray diffractogram peak list (purification option 1):

| **Peak No.** | **d/Å** | **°2θ (Cu-Kα₁ radiation) ± 0.1°** |
|---|---|---|
| 1 | 8.74 | 10.1 |
| 2 | 8.23 | 10.7 |
| 3 | 7.62 | 11.6 |
| 4 | 6.78 | 13.0 |
| 5 | 6.58 | 13.4 |
| 6 | 5.74 | 15.4 |
| 7 | 4.99 | 17.8 |
| 8 | 4.85 | 18.3 |
| 9 | 4.68 | 18.9 |
| 10 | 4.44 | 20.0 |
| 11 | 4.36 | 20.3 |
| 12 | 3.73 | 23.8 |
| 13 | 3.64 | 24.4 |
| 14 | 3.39 | 26.3 |
| 15 | 3.14 | 28.4 |

Powder X-ray diffractogram peak list (purification option 2):

| **Peak No.** | **d/Å** | **°2θ (Cu-Kα₁ radiation) ± 0.1°** |
|---|---|---|
| 1 | 8.75 | 10.1 |
| 2 | 8.23 | 10.7 |
| 3 | 7.62 | 11.6 |
| 4 | 6.80 | 13.0 |
| 5 | 6.60 | 13.4 |
| 6 | 5.75 | 15.4 |
| 7 | 4.99 | 17.7 |
| 8 | 4.86 | 18.2 |
| 9 | 4.69 | 18.9 |
| 10 | 4.44 | 20.0 |
| 11 | 4.37 | 20.3 |
| 12 | 3.74 | 23.8 |
| 13 | 3.64 | 24.4 |
| 14 | 3.39 | 26.2 |
| 15 | 3.14 | 28.4 |

Powder X-Ray Diffraction pattern and corresponding XRD data confirmed that compound "A7" is crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate.

### Example 2: Anti-cancer testing of compounds

Complete Response to Adjuvant Tepotinib Hydrochloride Hydrate in A Patient with Newly Diagnosed Disseminated Glioblastoma (GBM) Harboring MET Amplification
Here, we report complete radiographic response to tepotinib hydrochloride hydrate in a patient with newly diagnosed disseminated GBM harboring MET amplification. A 29-years-old male presented with progressive headache. MRI brain showed a large heterogeneously enhancing intraventricular mass with epicenter in the right lateral ventricular trigone and enhancing nodules in bilateral cerebellum. MRI spine showed multifocal enhancement along the periphery of the cervicothoracic spinal cord and cauda equina concerning for leptomeningeal disease. He underwent right temporal lobectomy and subtotal resection of the mass. His KPS was 90 post-surgery due to dizziness. Pathology was consistent with GBM, IDH wild-type, MGMT-unmethylated and MET amplification at 7q31.2. He completed proton craniospinal irradiation (CSI) at 3600 cGy followed by boost to the tumor bed with intensity modulated radiotherapy (IMRT) at 2400 cGy without concurrent temozolomide (TMZ) due to the large radiation field. He completed two cycles of adjuvant TMZ which was put on hold due to myelosuppression. He subsequently started tepotinib hydrochloride hydrate monotherapy at 1000 mg daily obtained on a compassionate IND. MRIs after one month of therapy showed resolution of areas of enhancement in the brain and spine. Specifically, MRI brain, cervical, thoracic, and lumbar spine showed near complete resolution of his contrast enhancing lesions and LMD following 1 cycle (=4 weeks) of tepotinib. He had grade 1 creatinine elevation and abdominal discomfort and dose was reduced to 500 mg daily after two cycles. Nineteen weeks after initiation of tepotinib hydrochloride hydrate, his complete response persists, and he remains clinically stable with mild chronic dizziness. Adjuvant tepotinib monotherapy was well-tolerated and conferred complete and durable control in the patient with disseminated, MET amplified GBM on latest MRI post cycle 11 (Fig. 5). Trials of targeted therapy in molecularly unselected GBM have been largely disappointing, however, this case demonstrates the promise of targeting MET using tepotinib or a pharmaceutically acceptable salt, solvate and/or hydrate thereof in GBM.

### Example 3: Effects of MET pathway inhibition and irradiation in syngeneic mouse models Materials and Methods

### Reagents and cell lines

Tepotinib was dissolved in DMSO and further diluted in cell culture medium. For *in vivo* studies, tepotinib was prepared with 20% solutol and 80% sodium acetate buffer (pH 5.5). Recombinant human HGF (GeneTex, Lucerne, Switzerland) was dissolved in Dulbecco's phosphate-buffered saline (PBS). The murine glioma cell lines SMA-497, SMA-540 and SMA-560 were obtained from Duke University Medical Center, Durham, NC, and GL-261 from the National Cancer Institute, Frederick, MD. The cells were cultured in Dulbecco's Modified Eagle Medium (DMEM) with 10% fetal calf serum (FCS) and 1% glutamine. Gene expression profiles for these cell lines have been generated previously (Ahmad *et al.,* 2014).

### Real-time polymerase chain reaction (RT-PCR)

cDNA generated by reverse transcription from 1 µg total RNA was subjected to RT-PCR. Relative gene expression was measured as described (Papa *et al.,* 2017) using a variation of the 2^(- delta delta CT) method (Livak and Schmittgen, 2001). Hypoxanthine-guanine phosphoribosyltransferase 1 (*Hprt1*) served as a reference gene. Mouse specific primer sequences were as follows: *Hprt1* fw: 5'- CCT AAG ATG AGC GCA AGT TGA A -3', rev: 5'- CCA CAG GAC TAG AAC ACC TGC TAA-3'; *Hgf* fw: 5'- TCA AAA TGT CAC CTA AAA CAA TCC - 3', rev: 5'- ACA AAC AAT ACA ACA GAA AAC ACC -3'; *Met* fw: 5'- TTT GGG GAA GTC TCA TTT TTG -3', rev: 5'- CGA TTT TCA GTT GGC TTT TG -3'.

### Immunoblot analysis

Lysates of cells or tumor specimens were prepared using radio-immunoprecipitation assay (RIPA) lysis buffer (pH 7.8) containing 25 mM Tris-HCl, 120 mM NaCl, 5 mM EDTA and 0.5% NP-40 supplemented with phenylmethylsulfonyl fluoride (Sigma Aldrich, St. Louis, MO), 200 mM sodium orthovanadate, 0.5 M sodium fluoride, protease inhibitor cocktail and phosphatase inhibitor cocktails 2 and 3 (Sigma Aldrich). Primary antibodies were as follows: rabbit anti-p-MET (Tyr1234/1235) (D26), mouse anti-MET (clone 25H2; both Cell Signaling Technology, Denvers, MA) or goat anti-actin (sc-47778; Santa Cruz Biotechnology, SC, Santa Cruz, CA). The membranes were exposed to HRP-conjugated secondary species-specific antibodies.

### Proteome profiler array

For the simultaneous determination of relative levels of mouse cytokines *in vivo,* the Mouse XL Cytokine Array Kit from R&D Systems was used (ARY028, Minneapolis, MN). Tissue lysates were prepared by electric homogenizer in ice-cold lysis buffer and supplemented with protease inhibitor cocktail, followed by centrifugation to remove cellular debris; 150 µg protein was used for each tumor sample and nitrocellulose membrane (control, monotherapies, combination therapy). To compare the proportion of protein levels in each tumor specimen, the quantification of pixel density of each spot of the array was performed using Image J (version 1.32j) software (National Institutes of Health, Bethesda, MD, http://rsb.info.nih.gov/ij/).

### Enzyme-linked immunosorbent assays (ELISA)

Mouse HGF was quantified with the Mouse HGF ELISA Kit (#EMHGF, Thermo Scientific, Frederick, MD) using serum-free supernatants of 4 x 10⁶ cells collected after 36 h.

### CRISPR/Cas 9 knockout of MET

Knockout clones were generated as described (Ran *et al.,* 2013; Machado *et al.,* 2019). Briefly, two *MET*-specific guide RNAs (5'-GAGAGCACGACAAATACGTA-3' and 5'-GTATCGGACAGAGTTTACCA-3') were cloned into pSpCas9(BB)-2A-GFP and lipofectamine (Thermo) was used to co-transfect both plasmids. After puromycin selection, single cells were sorted into 96 well plates using a BD FACSAria III. Knock-out was confirmed with RT-PCR and Sanger sequencing after expansion of clones.

### Animal studies

All experiments were conducted in accordance with the Swiss Cantonal Veterinary Office under the animal license permission numbers ZH-038 and ZH-098 according to guidelines of the Swiss federal law on animal protection. mRNA was prepared from untreated gliomas that started to render mice symptomatic or from non-tumor-bearing mouse brain. For treatment studies, 5,000 tumor cells were stereotactically implanted into the right striatum of six to 12-week-old immunocompetent VM/Dk mice, immunocompetent C57BL/6 mice or "non-leaky" immune deficient *Rag1-*/*-* (B6.129S7-*Rag1^{tm1Mom}*/J) mice, devoid of mature B and T cells, on a C57BL/6 background. Tepotinib was administered orally using gavage at 100 mg/kg. Local cranial radiotherapy was performed at day 10 after tumor implantation using a Gulmay 200 kV X-ray unit at 1 Gy/minute at room temperature (Schneider *et al.,* 2017). Neurological symptoms were assessed daily according to the cantonal veterinary office Zurich guidelines (grade 0: no visible impairment, grade 1: reduced activity, slight balance and coordination impairments, grade 2: reduced activity, 15% weight loss compared to peak weight, slight paralysis of left-handed legs, moderate signs of pain). For assessment of early histological changes, animals were euthanized when the first animal showed a grade 2 manifestation of disease progression, including neurologic symptoms. Total mRNA or protein lysates were prepared from 20-25 mg brain tissue collected at the time of sacrifice from the left and right hemispheres and the tumor.

### Histology and immunohistochemistry

Sections (8 µm thick) of tumor-bearing cryopreserved brains were prepared. The mean tumor size was determined by the largest tumor area in the horizontal plane on hematoxylin and eosin (H&E)-stained sections by multiplying the longest perpendicular diameters from a low-magnification (2.5x) image. Brain sections were immunostained with Ki-67 antibody (clone SP6, Epitomics, Burlingame, CA), anti-mouse CD31 antibody (clone MEC 13.3, BD Pharmingen, Franklin Lakes, NJ), anti-CD45 antibody (clone 30-F11; BioLegend), anti-CD3 (clone 17A2; BD Biosciences, San Jose, CA) or anti-CD11b (clone M1/70; BD Biosciences) and histofine simple stain mouse max pro anti-rabbit secondary antibody (Nichirei Biosciences, Tokyo, Japan) to determine proliferation. Anti-rat N-Histofine Simple Stain Mouse MAX PO (Nichirei Biosciences) and DAB chromogen were used to stain blood vessels. The percentage of Ki-67-positive tumor nuclei and the mean number of CD31-positive intratumoral vessels per high power field (×40 objective) were calculated using three randomly selected different microscopic fields of each section for three 3 mice per group. Alternatively, DAB-positive signals were quantified using the ImageJ software (https://imagej.nih.gov/) (Crowe and Yue, 2019).

### Statistical analysis

All *in vitro* and *in vivo* experiments reported here were performed in biological replicates, i.e., in independent experiments and with different passage numbers of cell lines. Statistical significance was assessed by two-sided unpaired t-test or two-way ANOVA with Bonferroni post-testing (GraphPad Prism). Tumor size was analyzed using the Mann-Whitney U non-parametric test. Survival was analyzed using a Kaplan-Meier survival plot followed by a log-rank (Mantel-Cox) test. Differences were considered statistically significant when the p value was below 0.05.

### Results

Mouse glioma cells expressed HGF and MET and responded to exogenous HGF with MET phosphorylation (p-MET). Glioma cell viability or proliferation were resistant to genetic or pharmacological MET inhibition using tepotinib or *met* gene knockout. MET inhibition failed to sensitize glioma cells to irradiation *in vitro.* In contrast, the combination of tepotinib with radiotherapy prolonged survival of orthotopic SMA-560 or GL-261 glioma-bearing mice compared with radiotherapy or tepotinib treatment alone, associated with suppression of glioma cell proliferation and delayed tumor growth. In the SMA-560 model, combination therapy suppressed a set of pro-inflammatory cytokines which were upregulated by irradiation alone and had been linked to poor outcome in glioblastoma. Synergy was lost when SMA-560 gliomas were established and treated in immunodeficient RAG-/- mice. In the GL-261 model, synergy was also lost in *Rag1-*/*-* mice, but, importantly, also when MET gene expression was disrupted in the tumor. In detail:

### Characterization of the HGF/MET axis in mouse glioma models in vitro

We first examined HGF and MET expression levels and constitutive MET activation in the GL-261, SMA-497, SMA-540 or SMA-560 mouse glioma models. All cell lines expressed *Hgf* and *Met* mRNA *in vitro* and *ex vivo. Ex vivo* tumor samples from all four models exhibited similar levels of *Hgf* mRNA which paralleled the levels in normal brain tissue. In contrast, *Met* mRNA expression was significantly upregulated in tumor tissues compared to brain tissue (Fig. 1A, 1B). *Hgf*mRNA expression and HGF protein release *in vitro* were highly correlated (r=0.998, p=0.002) among the four mouse cell lines, SMA-540 demonstrating the highest HGF expression (Fig. 1C). Constitutive MET phosphorylation was detected in SMA-497 and in SMA-560 cells. All cell lines accumulated p-MET in response to exogenous HGF (Fig. 1D). GL-261 showed the lowest basal MET activation status based on the microarray-based transcriptional profiling of MET-driven genes. Next, we confirmed that exposure of SMA-497 and SMA-560 cells to the MET inhibitor, tepotinib, suppressed MET phosphorylation in a time- and concentration-dependent manner (Fig. S1A). Yet, glioma cell lines were resistant to inhibitory effects of tepotinib at concentrations up to 1 µM in acute growth inhibition or clonogenicity assays (Fig. S1B, S1C).

### Irradiation induced HGF/MET signaling, but no radio-sensitization by inhibition of HGF/MET signaling in vitro

There were no major changes in *Hgf* or *Met* mRNA expression upon irradiation in GL-261, SMA-497 or SMA-540 cells. In contrast, there was an irradiation dose-dependent induction of *Hgf* and *Met* mRNA expression in SMA-560 cells (Fig. S2A), which translated into tepotinib-sensitive MET phosphorylation (Fig. S2B). Yet, at concentrations known to specifically inhibit MET phosphorylation (Fig. S1A), tepotinib failed to sensitize glioma cells to the inhibitory effects of irradiation *in vitro.* To rule out that this negative effect was merely the result of low availability of HGF in culture, similar experiments were conducted in the presence of exogenous HGF, but again no sensitization to irradiation by tepotinib became apparent (Fig. S2C).

### Tepotinib inhibits basal and irradiation-induced activation of murine glioma cell invasiveness in vitro

Next, we evaluated the motogenic activity of HGF/MET in response to irradiation *in vitro.* Low-dose irradiation (2 Gy) increased invasiveness in SMA-540 and SMA-560 cells whereas high-dose irradiation (8 Gy) reduced invasiveness in SMA-497 and SMA-560 cells. Tepotinib suppressed invasiveness in all cell lines without or with irradiation, except in low dose-irradiated SMA-540 cells. Similar trends were observed in spheroid invasion assays (Szabo *et al.,* 2016) using SMA-560 cells.

### Synergistic growth inhibition of murine SMA-560 gliomas by radiotherapy and tepotinib in vivo

Based on high *Met* gene expression and constitutive MET phosphorylation in the tumor tissue (Fig. 1B), the SMA-560 model was selected to assess the effects of radiotherapy in combination with pharmacologic inhibition of MET by tepotinib *in vivo.* We first confirmed target inhibition by tepotinib by measuring p-MET levels by immunoblot *ex vivo*: p-MET was detected in the tumor and in the tumor-bearing right hemisphere. It was induced by irradiation, but no p-MET was detected in animals treated with tepotinib (Fig. 2A). Neither tepotinib alone nor radiotherapy alone had a significant effect on survival, but their combination resulted in a strong synergistic prolongation of survival from around 20 days without or with monotherapy to around 60 days with the combination. In the control and in the radiotherapy groups, all mice were euthanized because of symptoms related to tumor growth. In contrast, one mouse in the tepotinib group and three mice in the combination group were alive and free from tumor at day 80 (Fig. 2B). To gain insight in the mode of action mediating synergy *in vivo,* we performed tissue analysis using morphological and immunohistochemical assessments from mice per a randomization list when the first mouse in any group became symptomatic. Animals in all treatment groups exhibited a ~30-40% reduction in tumor volume relative to untreated controls. Yet, while the percentage of Ki-67-stained nuclei remained unaffected in both monotherapy groups, there was a strong decrease of Ki-67-positive cells in the combination group (Fig. 2C). No such effect of the combination was observed when the same treatments were administered acutely *in vitro.* SMA-560 cells give rise to highly vascularized tumor reflected by high density of CD31-positive vessels (Ahmad *et al.,* 2014). Here, we observed a reduction in CD31 immunoreactivity in both monotherapy arms (1.8-fold) and more so in the combination arm (2.8-fold) (Fig. 2D).

### Cellular and molecular mechanisms mediating synergistic growth inhibition of experimental gliomas by irradiation and tepotinib

Since we observed synergy of growth inhibition *in vivo,* but not in *vitro,* we concluded that the microenvironment might contribute to the therapeutic effect. Accordingly, we assessed differences in host cell infiltration in tumors from the four treatment groups, but observed no consistent differences at early timepoints, that is, when the first mice became symptomatic. We then compared the cytokine profiles in tumor tissue lysates *ex vivo* among the 4 treatment arms. We first noted that almost all cytokines were induced rather than decreased by any therapeutic measure; only tepotinib monotherapy reduced the levels of some cytokines, whereas irradiation alone produced the most prominent induction of cytokines (Fig. 3). The only cytokine that was induced by the combination, but not by either monotherapy alone, was the WNT1-inducible signaling pathway protein (WISP/CCN4). The likely most relevant pattern was an induction upon irradiation that was attenuated by tepotinib. This group of molecules included mediators of angiogenesis and pro-inflammatory cell adhesion molecules including VEGF, intercellular adhesion molecule 1 (ICAM-1 / CD54), serpinE1/PAI-1, P-selectin and the matrix metalloproteinases (MMP) -2 and -9, the antiangiogenic and anti-tumorigenic factor SerpinF1/PEDF, mediators of the innate immune response such as lipocalin-2, the colony stimulating factor 1 M-CSF and the innate pattern recognition molecules pentraxin 2/3 and C-reactive protein (CRP, pentraxin 1), pro-inflammatory cytokine IL-1a/IL-1F1 and CXCL10; the anti-inflammatory cytokines IL-10 and IL-11, the member of the notch/delta/serrate protein family pref-1/DLK-1/FA1, and low-density lipoprotein receptor (LDL-R). Among these differentially regulated factors, IL1A gene expression is associated with poor prognosis in glioblastoma patients.

### Synergistic suppression of glioma growth by irradiation and tepotinib requires adaptive immunity and MET expression in the tumor

To dissociate tumor microenvironmental *versus* tumor-autonomous mechanisms in mediating the synergistic response of experimental gliomas to the combination of radiotherapy and tepotinib, we selected a two-fold approach and studied a second model: we explored the combination therapy in immunodeficient animals, and we disrupted the MET gene in GL-261 glioma cells. In contrast to wildtype cells, MET-deficient GL-261 did not respond with MET phosphorylation upon exogenous HGF exposure but had no growth disadvantage *in vitro* as determined by measures of cell doubling time, cell cycle progression or viability *in vitro. Ex vivo* analysis demonstrated that MET-deficient tumors displayed similar properties of growth in immunocompetent syngeneic mice and comparable immune cell infiltration. Vessel density was significantly reduced in MET deficient tumors, which corresponded to the pattern seen with tepotinib treatment (Fig. 2C). First, we noted that the synergistic effect of combination therapy was strongly attenuated when SMA-560 cells were grown in RAG-/- mice (Fig. 4A). In the GL-261 model, combination therapy also resulted in a prominent survival prolongation when tumors were generated and treated in wildtype mice. There were approximately 50% long-term survivors in the co-treatment group (Fig. 4B). The long-term survivor mice were rechallenged with GL-261 cells at 90 days and all 4 animals remained asymptomatic until termination of the experiment after another 50 days (Fig. 4C). When the same experiment was conducted in RAG-/- mice, irradiation retained its activity, but synergy with tepotinib was lost (Fig. 4D). Finally, when MET was disrupted, irradiation was still as active as against wildtype tumors, but synergy was also lost in this model (Fig. 4E). Comparative survival data are compiled in Fig. 4F.

### Discussion

Although synergistic effects of MET inhibition and irradiation have previously been proposed to be mediated by interference with the cytoprotective role of the MET pathway in the context of DNA repair in human glioma, gastric adenocarcinoma and lung carcinoma cells (Welsh *et al.,* 2009; De Bacco *et al.,* 2011; Medová *et al.,* 2012), such mechanisms were unlikely to be operating in our models since there was no synergy between irradiation and pharmacological MET inhibition *in vitro* (Fig. S2). Conversely, we observed strong synergy of irradiation and tepotinib in two syngeneic immunocompetent glioma models *in vivo* (Fig. 2, 4), which was not predicted by the *in vitro* data. Compared with either treatment alone, combination treatment induced an early suppression of proliferation and of angiogenesis in the SMA-560 model (Fig. 2). Inhibition of angiogenesis may be a consequence of suppression of irradiation-induced increases in angiogenic molecules such as VEGF, MCSF, MMP2 or MMP9 upon co-treatment with tepotinib (Fig. 3). Glioblastoma develops radiation resistance by multiple adaptive molecular strategies (Bao *et al.,* 2006; De Bacco *et al.,* 2011; Squatrito and Holland, 2011). Altogether, we report the attenuation by tepotinib of expression of several irradiation-induced cancer-related inflammation and immunoregulatory cytokines, as a potential mechanism by which tumor growth is delayed by tepotinib when combined with radiotherapy. Complementary studies exploring the efficacy of combination therapy either in immunodeficient mice or in mice carrying tumors with disrupted MET gene expression confirmed that synergy requires at least two mechanisms, first, expression of MET in the tumor, second, a functional immune system (Fig. 4). Our preclinical data would therefore suggest that MET pathway inhibition in human glioblastoma could be favorably explored in combination with radiotherapy.

### References

Ahmad M, Frei K, Willscher E, Stefanski A, Kaulich K, Roth P, et al. Journal of neuropathology and experimental neurology 2014; 73(11): 1062.
Bao S, Wu Q, McLendon RE, Hao Y, Shi Q, Hjelmeland AB, et al. Nature 2006; 444(7120): 756.
Crowe AR, Yue W. Bio-protocol 2019; 9(24).
De Bacco F, Luraghi P, Medico E, Reato G, Girolami F, Perera T, et al. Journal of the National Cancer Institute 2011; 103(8): 645.
Livak KJ, Schmittgen TD. Methods (San Diego, Calif) 2001; 25(4): 402.
Machado RAC, Schneider H, DeOcesano-Pereira C, Lichtenstein F, Andrade F, Fujita A, et al. Sci Rep 2019; 9(1): 3952.
Medová M, Aebersold DM, Zimmer Y. International journal of cancer 2012; 130(3): 728.
Papa E, Weller M, Weiss T, Ventura E, Burghardt I, Szabó E. Cell death & disease 2017; 8(12): 3210.
Ran FA, Hsu PD, Wright J, Agarwala V, Scott DA, Zhang F. Nat Protoc 2013; 8(11): 2281.
Schneider H, Lohmann B, Wirsching HG, Hasenbach K, Rushing EJ, Frei K, et al. Oncotarget 2017; 8(50): 87124.
Squatrito M, Holland EC. Cancer research 2011; 71(18): 5945.
Szabo E, Schneider H, Seystahl K, Rushing EJ, Herting F, Weidner KM, et al. Neuro-oncology 2016; 18(9): 1242.
Welsh JW, Mahadevan D, Ellsworth R, Cooke L, Bearss D, Stea B. Radiation oncology (London, England) 2009; 4: 69.

## Claims

1. A method for long-term treatment of a glioblastoma in a human subject in need thereof, comprising administering an effective amount of a cMET inhibitor to the subject for at least 10 weeks in a line of treatment, wherein the cMET inhibitor is 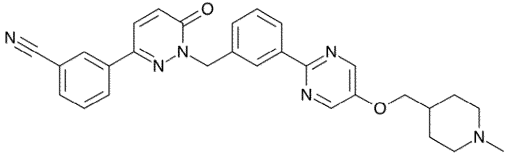 or a pharmaceutically acceptable salt, solvate and/or hydrate thereof, and wherein the cMET inhibitor is not administered in combination with chemotherapy in the same line of treatment.

2. The method of claim 1, wherein the cMET inhibitor is selected from the group of 3-(1-{3-[5-(1-methyl-piperidin-4-ylmethoxy)-pyrimidin-2-yl]-benzyl}-6-oxo-1,6-dihydro-pyridazin-3-yl)-benzonitrile, 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate, 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate, and crystalline modification H2 of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride monohydrate.

3. The method of any one of the preceding claims, wherein disseminated glioblastoma, glioblastoma harboring MET amplification, or a combination thereof, is treated.

4. The method of any one of the preceding claims, wherein glioblastoma harboring IDH wild-type, MGMT-unmethylated or MET amplification at 7q31.2, or a combination thereof, is treated.

5. The method of any one of the preceding claims, wherein the administration of the cMET inhibitor results in one or more clinical benefits, which are selected from the group of complete response, increase in the likelihood of complete response, reduction in the likelihood of development of drug resistance, extension in time to tumor progression, and extension in time to tumor recurrence; preferably complete response.

6. The method of any one of the preceding claims, wherein the cMET inhibitor is administered for at least about 11-52 weeks.

7. The method of any one of the preceding claims, further comprising administering radiotherapy in the same line of treatment; wherein, optionally, the radiotherapy is administered as single irradiation dose or after first administration of the cMET inhibitor, or a combination thereof.

8. The method of claim 7, wherein the radiotherapy is administered within 96 hours after first administration of the cMET inhibitor, preferably within 36 hours.

9. The method of any one of the preceding claims, wherein the cMET inhibitor is administered in a second-line treatment or higher line of treatment of the glioblastoma.

10. The method of any one of the preceding claims, further comprising administering a treatment selected from the group of surgery, radiotherapy, chemotherapy, and a combination thereof, prior to first administration of the cMET inhibitor; wherein, optionally, the surgery or radiotherapy, or the combination thereof, is administered first, followed by chemotherapy, prior to the first administration of the cMET inhibitor.

11. The method of any one of claims 1-8, wherein the cMET inhibitor is administered in a first-line treatment of the glioblastoma.

12. The method of claim 11, further comprising administering a treatment selected from the group of surgery, radiotherapy, chemotherapy, and a combination thereof, after first administration of the cMET inhibitor.

13. The method of any one of claims 7-12, wherein the radiotherapy is administered; wherein, optionally, the radiotherapy is proton irradiation.

14. The method of any one of claims 7-13, wherein the radiotherapy comprises about 3000-4000 cGy, optionally followed by boost to tumor bed at 2000-3000 cGy.

15. The method of any one of claims 1-6, wherein the cMET inhibitor is administered as monotherapy.
